# EUROPÄISCHE PATENTSCHRIFT

(11) **EP 0 714 903 B1**
(45) Veröffentlichungstag und Bekanntmachung des Hinweises auf die Patenterteilung: **15.05.2002**
(21) Anmeldenummer: 95115588.6
(22) Anmeldetag: 04.10.1995
(51) Int. Cl.: C07H 13/04, C07H 15/04, A61K 31/70

(54) **Kohlenhydratkonjugate als Inhibitoren der Zelladhäsion**
Carbohydrate conjugates as cell adhesion inhibitors
Carbohydrat conjugués utilisés comme inhibiteurs d'adhésion cellulaire

(30) Priorität: 10.10.1994 DE 4436164
(43) Veröffentlichungstag der Anmeldung: 05.06.1996
(73) Patentinhaber: Glycorex AB, 223 70 Lund (SE)
(72) Erfinder: Kretzschmar, Gerhard, Dr., D-65760 Eschborn (DE); Schmidt, Wolfgang, Dr., D-65929 Frankfurt (DE); Sprengard, Ulrich, DCh., D-55126 Mainz (DE); Bartnik, Eckart, Dr., D-65205 Wiesbaden-Delkenheim (DE); Seiffge, Dirk, Dr., D-55246 Mainz-Kostheim (DE); Kunz, Horst, Prof. Dr., D-55127 Mainz (DE)
(74) Vertreter: Bjerre, Nils B.J.

(56) Entgegenhaltungen:
- EP-A- 0 601 417
- EP-A- 0 606 925
- WO-A-92/02527
- WO-A-93/24506
- WO-A-94/00477
- J. CARBOHYD. CHEM., Bd. 12, Nr. 8, 1993 Seiten 1203-1216, XP 000566815 HASEGAWA ET AL 'synthetic studies on sialoglycoconjugates 52:'
- J. BIOL. CHEM. VOL.265 (14); PP.7864-71, Bd. 265, Nr. 14, 1990 (US), Seiten 7864-7871, XP 000566448 LEE R T ET AL 'Binding characteristics of galactoside-binding lectin (galaptin) from human spleen'
- CARBOHYDRATE RESEARCH, Bd. 236, 1992 AMSTERDAM, NL, Seiten 349-356, OHBAYASHI 'Production of Monoclonal antibodies against Oligosaccharides coupled to Protein'
- ANGEW. CHEM. (INT. ED.), Bd. 33, Nr. 1, 1994 WEINHEIN,DE, Seiten 101-102, VON DEM BRUCH 'Synthesis of N-Glycopeptide Clusters.....'
- CARBOHYDR. RES. , Bd. 219, 1991 UNIV. ORLEANS;FAC. SCI.; ORLEANS; F-45067; FR. (FR), Seiten 71-90, RIO S ET AL 'Synthesis of glycopeptides from the carbohydrate-protein linkage region of proteoglycans'
- ANGEW. CHEM., Bd. 33, Nr. 20, November 1994 WEINHEIM,DE, Seiten 2096-2098, W. STAHL ET AL 'Synthesis of deoxy Sialyl Lewisx Analogues, Potential Selectin Antagonists'

## Beschreibung

Die Erfindung betrifft neue Konjugate von Sialyl-Lewis-X (SLeX) und Sialyl-Lewis-A (SLeA), mit verbesserter Wirkung als Inhibitoren der Zelladhäsion, ein Verfahren zur Herstellung dieser Verbindungen sowie deren Verwendung als pharmakologische Wirkstoffe und als Diagnostika und Arzneimittel, welche diese Konjugate enthalten.

Die Zirkulation von Blutzellen wie z.B. Leukozyten, Neutrophilen, Granulozyten und Monozyten ist auf molekularer Ebene ein vielstufiger, sehr komplexer Prozess, der nur in Teilschritten bekannt ist (Review: T. A. Springer, Cell 76, 301-314, 1994).

Jüngste Forschungsergebnisse zeigten, daß die in der Immunüberwachung entscheidende Rezirkulation der Lymphozyten sowie die Lokalisierung von Neutrophilen und Monozyten an Entzündungsherden sehr ähnlichen molekularen Mechanismen gehorchen. So kommt es bei akuten und chronischen Entzündungsprozessen zur Adhäsion der Leukozyten an Endothelzellen und Auswanderung in den Entzündungsherd und in die sekundären lymphatischen Organe.

An diesem Vorgang sind zahlreiche spezifische Signalmoleküle wie z.B. Interleukine, Leukotriene und Tumornekrosefaktor (TNF), an deren G-Protein gekoppelte Rezeptoren und insbesondere gewebespezifische Zelladhäsionsmoleküle beteiligt, die eine genau kontrollierte Erkennung der Immun- und Endothelzellen gewährleisten. Zu den wichtigsten hierbei beteiligten Adhäsionsmolekülen, die im folgenden als Rezeptoren bezeichnet werden sollen, gehören die Selektine (E-, P- und L-Selektine), Integrine und die Mitglieder der Immunglobulin-Superfamilie.

Die drei Selektinrezeptoren bestimmen die Anfangsphase der Leukozytenadhäsion. E-Selektin wird auf Endothelzellen einige Stunden nach der Stimulierung beispielsweise durch Interleukin-1 (IL-1) oder Tumornekrosefaktor α (TNF-α) exprimiert, während P-Selektin in Blutplättchen und Endothelzellen gespeichert wird und nach Stimulierung durch Thrombin, Peroxidradikale oder Substanz P u.a. auf den Zelloberflächen präsentiert wird. L-Selektin wird dauerhaft auf Leukozyten exprimiert.
Es wird heute generell anerkannt, daß die Tetrasaccharide Sialyl Lewis-X (SLeX) und Sialyl Lewis-A (SLeA), die als Teilstrukturen von Glycosphingolipiden und Glycoproteinen auf Zellmembranen vorkommen, als Liganden für alle drei Selektinrezeptoren fungieren können. (Review: A. Giannis, Angew. Chem. 106, 188, 1994):

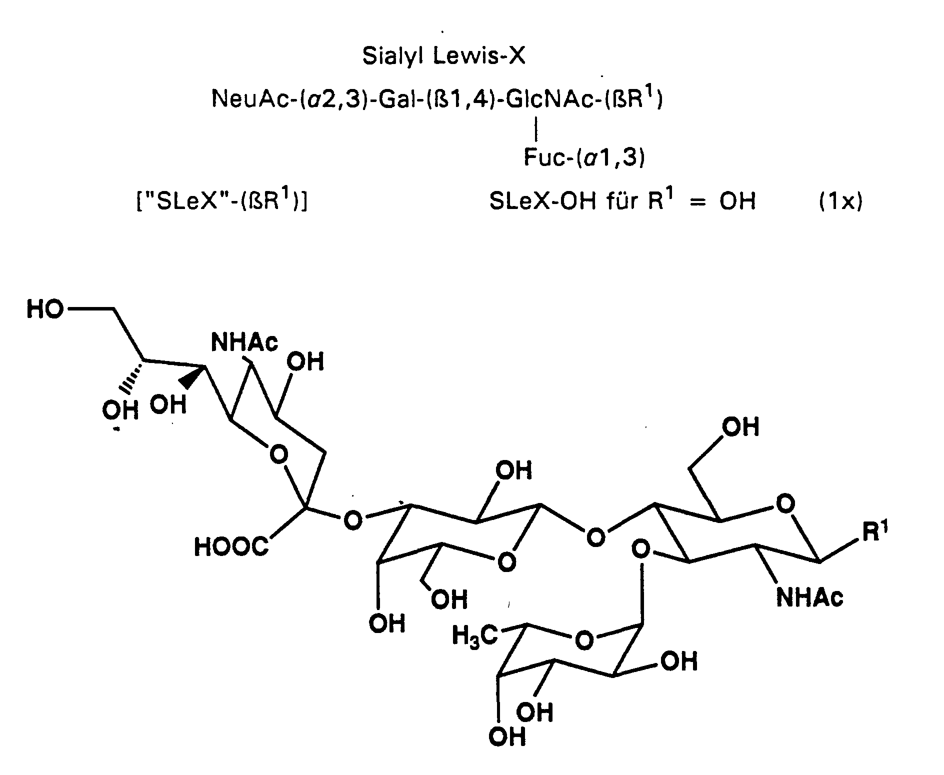

Die regioisomere Verbindung Sialyl Lewis-A ist dem X-Typ eng verwandt und bindet mit vergleichbarer Affinität an Selektinrezeptoren. Der A-Typ geht aus dem X-Typ durch einfache Vertauschung der "Seitenketten" am zentralen N-Acetylglucosamin-Baustein hervor:

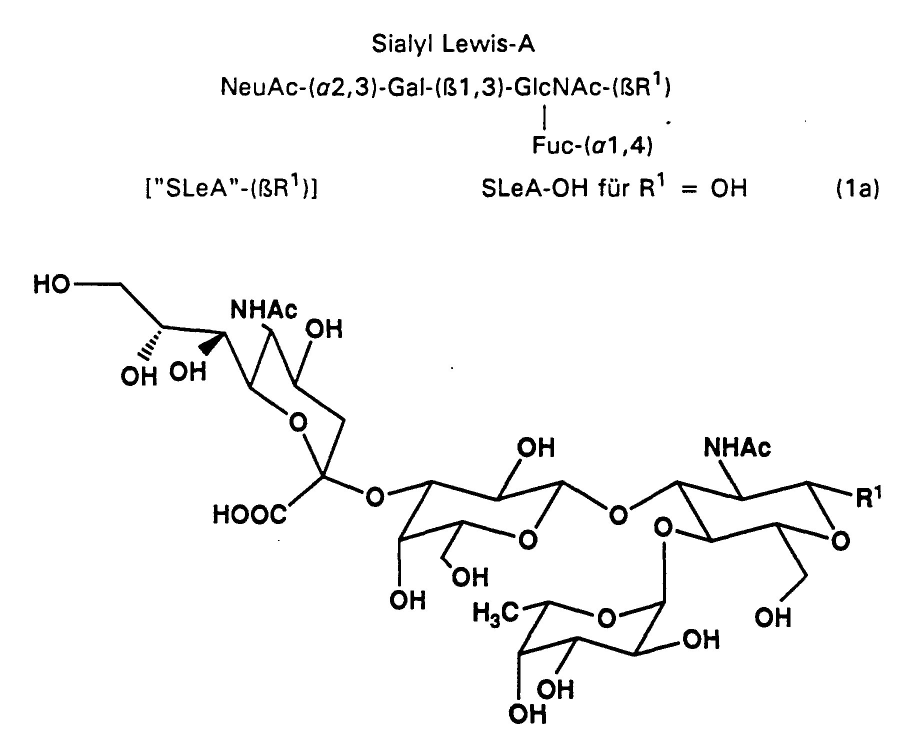

Der Verlauf einer Reihe von akuten und chronischen Erkrankungen wird durch die übermäßige Adhäsion von Leukozyten und deren Infiltration in das betroffene Gewebe ungünstig beeinflußt. Dazu zählen beispielsweise Rheuma, Reperfusionsverletzungen wie myokardiale Ischämie/Infarkt (MI), akute Lungenentzündung nach operativem Eingriff, traumatischer Schock und Schlaganfall, Psoriasis, Dermatitis, ARDS (Atemnotsyndrom bei Erwachsenen) sowie die nach chirurgischen Eingriffen (Beispiel Angioplastie) auftretende Restenose.

Ein vielversprechender therapeutischer Ansatz ist deshalb der Versuch, die Tetrasaccharide SLeX/A in verschiedenen Darreichungsformen oder Mimetika derselben mit modifizierter Struktur als Antagonisten zur Modulierung oder Unterbindung einer übermäßigen Leukozytenadhäsion einzusetzen und zur Linderung bzw. Heilung genannter Erkrankungen einzusetzen.

Der natürliche Ligand mit der Struktur von SLeX wurde schon erfolgreich in Tierversuchen bei P-Selektin abhängigen Lungenverletzungen (M. S. Mulligan et al., Nature 1993, 364, 149) und bei myokardialen Reperfusionsverletzungen (M. Buerke et al., J. Clin. Invest. 1994, 93, 1140) verwendet. In ersten klinischen Prüfungen bei akuter Lungenentzündung soll die Verbindung in einer Dosis von 1 bis 2 Gramm pro Tag und Patient eingesetzt werden (Mitteilung der Firma Cytel Corp./La Jolla (CA.) beim 2. Glycotechnology Meeting/CHI in La Jolla/USA am 16.-18. Mai 1994). Diese hohe Wirkstoffdosis steht im Einklang mit der bekanntermaßen schwachen Affinität der natürlichen SLeX/A-Liganden zu den Selektinrezeptoren. So inhibiert SLeX in allen bekannten in vitro-Testsystemen die Zelladhäsion an Selektinrezeptoren erst bei einer relativ hohen Konzentration im Bereich von IC₅₀ = 1 bis 3 mM).

In einigen Publikationen wurde inzwischen über Bemühungen berichtet, durch strukturelle Variation des Liganden zu fester bindenden Antagonisten zu gelangen. Die Variation der für die Struktur-Wirkungs-Beziehung bislang als entscheidend angesehenen Fucose- und Neuraminsäurebausteine (B. K. Brandley et al., Glycobiology 1993, 3, 633 und M. Yoshida et al., Glycoconjugate J. 1993, 10, 3) erbrachte jedoch keine signifikant verbesserten Inhibitionswerte. Allein bei Variation des Glucosaminbausteins (Ersatz von GlcNAc durch Glucose und Azido- sowie Aminogruppen in der 2-Position von GlcNAc) ließ sich eine signifikant erhöhte Affinität an den E-Selektin-Rezeptor erreichen. Die IC₅₀-Daten dieser durch vollkommenen Neuaufbau herstellbaren Verbindungen sollen für die Hemmung der Adhäsion von HL-60 und U-937 Zellen bei 0.12 mM (gegenüber 1.2 bis 2.0 mM für SLeX) bei E-Selektin liegen. Von Nachteil ist hingegen, daß die Bindung an L- und P-Selektine mit >5 mM stark beeinträchtigt wird (Dasgupta et al., Posterpräsentation der Firma Glycomed Inc. anläßlich der Tagung in La Jolla in 5/94).

In einem anderen in vitro-Testsystem, bei dem unter Umkehrung der natürlichen physiologischen Verhältnisse das lösliche Rezeptorkonstrukt E-Selektin-IgG (anstelle des entsprechenden Rezeptorkonstruktes in immobilisierter Form, was eher mit der natürlichen Situation auf Endothelzellen vergleichbar wäre) an den immobilisierten Liganden bindet und von potentiellen Inhibitoren verdrängt wird, wurde in dem System E-Selektin-IgG/immobilisiertes BSA-SLeA für den Liganden mit in 2-Position deacetyliertem N-Acetylglucosamin eine 36fach höhere Affinität gefunden.

Abgesehen von einer eingeschränkten Vergleichbarkeit dieses artifiziellen Testsystems mit der Situation in vivo, also der Inhibierung der Adhäsion von Zellen, die die natürlichen Liganden SLeX/A exprimieren, bleibt dieses Ergebnis auf den E-Selektinrezeptor beschränkt, denn mit dem P-Selektinrezeptor wurden nur schwache Inhibitionseffekte bei Inhibitorkonzentrationen von ca. 1 mM gefunden (R. M. Nelson et al., J. Clin. Invest. 1993, 91, 1157).

Der Stand der Technik zur Bindungsaffinität modifizierter SLeX/A-Strukturen an Selektine wird in Pharmacochem. Libr. 1993, 20 (Trends in Drug Research), Seiten 33-40 referiert.

Modifizierte Liganden vom SLeX/A-Strukturtyp, die sich vorwiegend vom Lactose- und vom Lactosamin-Grundgerüst ableiten und u.a. als potentielle Selektinantagonisten eingesetzt werden könnten, werden in mehreren Patentanmeldungen beansprucht, insbesondere in den internationalen Veröffentlichungen
WO 91/19501, WO 91/19502, WO 92/02527, WO 93/10796, WO 94/00477, WO 92/18610, WO 92/09293, WO 92/07572, WO 92/16640, WO 92/19632, WO 93/17033, WO 93/23031, WO 92/22301, WO 92/22563, WO 92/22564, WO 92/22565, WO 92/22661, WO 92/22662, WO 93/24505, WO 93/24506.

SLeX/A-Derivate oder Mimetika mit deutlich verbesserter Affinität an den E-Selektin- und an den P-Selektin-Rezeptor in vitro wurden noch nicht beschrieben. Bemerkenswert sind dagegen Hinweise, daß multivalente Liganden eine höhere Bindungsaffinität gegenüber monovalenten Liganden haben könnten: So bindet ein enzymatisch hergestelltes, komplex aufgebautes Nonasaccharid als potentiell divalenter Ligand 5mal besser (IC₅₀ = 0.4 mM) an E-Selektin als der monomere SLeX-Ligand. Bei näherer Analyse stellt dieser Wert allerdings keinen überzeugenden Multivalenzeffekt dar: Berücksichtigt man, daß der IC₅₀-Wert pro Ligand gerechnet eigentlich nur 0.8 mM beträgt, so wurde tatsächlich keine signifikante Verbesserung erreicht (S. A. DeFrees et al., J. Am. Chem. Soc. 1993, 115, 7549).

Eine weitere Möglichkeit zur mehrfachen Präsentation der SLeX/A-Liganden besteht in der Einführung (co)polymerisierbarer Seitenketten oder in der Anbindung einer geeigneten SLeX-Vorstufe an ein multifunktionelles Polymer. Die erste Variante führt zu artifiziellen Polymerkonjugaten, beispielsweise zu Polyacrylamiden, die aufgrund ihrer physiologischen Unverträglichkeit nicht als Arzneimittel geeignet sind. In der Literatur wurde dieses Vorgehen für Polyacrylamidkonjugate des Lewis-X-Trisaccharids beschrieben (S.-I. Nishimura et al. Macromolecules 1994, 27, 157). Das Verfahren läßt sich auch auf SLeX, SLeA und Analoga, bei denen die Sialinsäure durch Sulfat ersetzt wurde, übertragen (E.Nifantév, Vortrag Glycotechnology Meeting La Jolla, 16.-18. Mai 1994).

Die zweite Variante, bei der SLeX-Derivate mit einem reaktiven Polymer zu multifunktionellen, biokompatiblen und physiologisch verträglichen Polymerkonjugaten umgesetzt werden, wird in der EP 0 601 417 beschrieben. In dieser Veröffentlichung wird auch der Stand der Technik zu Kohlenhydrat-Polymerkonjugaten ausführlich referiert.

Ein inhärenter Nachteil dieser Kohlenhydrat-Polymerkonjugate bezüglich ihrer Verwendbarkeit als Arzneimittel liegt in der Polymernatur derartiger Wirkstoffe:

Bei jedem Syntheseansatz wird ein neuer Produkttyp erhalten, der durch eine abweichende Molekulargewichtsverteilung und durch eine variable Belegungsdichte des am Polymer gebundenen Kohlenhydratliganden gekennzeichnet ist.

In der WO 94/00477 wird vorgeschlagen, multivalente Verbindungen durch reduktive Aminierung der Oximaddukte von Liganden des Strukturtyps (1), die als freie Oligosaccharide (d.h. R¹ = OH) vorliegen, mit Peptiden oder Proteinen herzustellen. Dieses Verfahren hat jedoch gravierende Nachteile, da der SLeX-Ligand durch die Ringöffnung des ersten Kohlenhydratbausteins am reduzierenden Ende (GlcNAc oder Glc) stark modifiziert wird. Außerdem sind wie bei den genannten Polymerkonjugaten nur ungenau definierte und nicht charakterisierbare Gemische zu erwarten. Dies zeigt gerade das in der WO 94/00477 hypothetisch beschriebene Beispiel, in dem unter Einsatz des 10.000fachen Überschusses (1 mmol) an wertvollem Oligosaccharid eine Ankopplung an das Tripeptid Lys-Tyr-Lys im analytischen Maßstab von 0.1 µmol Tripeptid Lys-Tyr-Lys vorgeschlagen wird. Nach Durchführung analytischer Trennverfahren wird die Möglichkeit einer Analyse mittels Massenspektroskopie erwähnt, wonach Gemische mono-, di- und trivalenter Kohlenhydratkonjugate erwartet werden.

Wünschenswert sind deshalb niedermolekulare Verbindungen mit eindeutiger Summenformel und definiertem Molekulargewicht sowie mit deutlich gesteigerter Rezeptoraffinität. Wünschenswert sind insbesondere Verbindungen, die allen genannten Anforderungen gleichzeitig genügen und darüber hinaus in effizienten Syntheseverfahren im präparativen Maßstab (Gramm-Mengen) herstellbar sind.

Aufgabe der Erfindung ist es, niedermolekulare Kohlenhydratrezeptorblocker, ein einfaches Verfahren zu deren Herstellung sowie aus diesen hergestellte Arzneimittel bereitzustellen, die den genannten Anforderungen genügen und insbesondere ein definiertes Molekulargewicht aufweisen.

Diese Aufgabe wird erfindungsgemäß gelöst durch die einfache, in wenigen Schritten und in hohen Ausbeuten durchführbare Kupplung von Oligosaccharide mit SLeX/A-Strukturen an mono- und trifunktionelle Vorstufen zu neuen Kohlenhydratkonjugaten. Der wertvolle Oligosaccharidbaustein kann stöchiometrisch oder in geringem Überschuß von 1 bis 10 mol% pro Ankergruppe und bevorzugt in ungeschützter Form eingesetzt werden.

Überraschenderweise werden bei dem erfindungsgemäßen Syntheseweg zu trivalenten Kohlenhydratkonjugaten nur geringe Mengen der divalenten Nebenprodukte gebildet, die sich leicht von den gewünschten Hauptprodukten abtrennen lassen.

Weiterhin überraschend ist der Befund, daß die neuen Konjugate stärker an E- und P-Selektine binden als der natürliche Ligand der Formel (1). Die agonistische und antagonistische Wirkung der erfindungsgemäßen Verbindungen läßt sich zur Vorbeugung, Therapie und Diagnose von Erkrankungen einsetzen, die durch eine übermäßige Zell-Zell-Adhäsion gekennzeichnet sind.

Die Erfindung betrifft somit eine Verbindung der Formel I,

Z-Y-(CH₂)ₙ-[NH(CO)]ₚ-R² I,

worin bedeuten
- Z: Sialyl-Lewis-X oder Sialyl-Lewis-A
- Y: Sauerstoff oder NH(CO), das in 1-Position mit Z verknüpft ist, und
- R²: einen Aminosäure- oder Oligopeptidrest von bis zu 6 Aminosäuren, einen lipophilen Rest aus aliphatischen oder cycloaliphatischen Bausteinen,
eine Kombination aus aliphatischen und heterozyklischen Bausteinen oder einen Triphenylmethanfarbstoff, wobei
- für: Y = Sauerstoff
- p: 1 und
- n: eine ganze Zahl von 2 bis 10 und
- für: Y = NH(CO) und p = 0
- n: für eine ganze Zahl von 0 bis 10, und
- für: Y = NH(CO) und p = 1
- n: eine ganze Zahl von 1 bis 10 bedeuten und
die Verbindung ein definiertes Molekulargewicht aufweist.

Vorzugsweise ist R² eine Gruppe der Formel (IA), 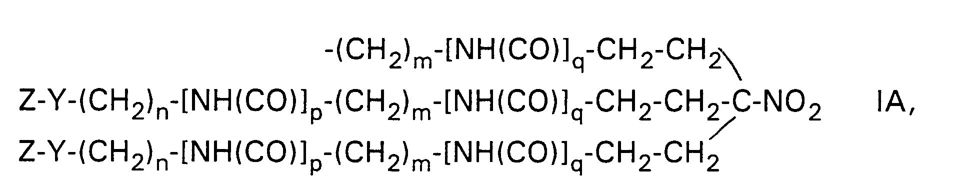 wobei
- m: eine ganze Zahl von 0 bis 10 ist und
- p und q: 0 oder 1 sein können mit der Maßgabe, daß
- für m = 0: entweder p oder q gleich 0 ist.

Diese bevorzugten Ausgestaltungen von Verbindung (I) (mit R² = IA) werden im folgenden Verbindung (II), 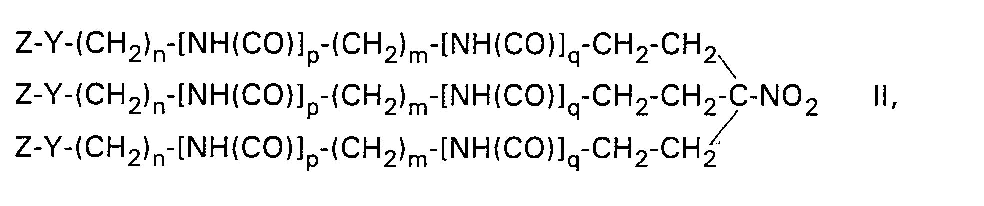 genannt.

Die vorliegende Erfindung betrifft ferner ein Verfahren zur Herstellung einer Verbindung (I), das sich dadurch auszeichnet, daß eine Verbindung der Formel (III),

Z-O-(CH₂)ₙ-NH₂ III,

eine Verbindung der Formel (IV),

Z-NH₂ IV,

oder eine Verbindung der Formel (V),

Z-NH(CO)-(CH₂)ₙ-NH₂ V,

in welchen Z und n die genannten Bedeutungen haben, mit einer Verbindung dei Formel (VI),

X(CO)R² VI,

in welcher
- X: Hydroxyl oder eine carboxylaktivierende Abgangsgruppe und
- R²: die genannten Bedeutungen hat,
umgesetzt wird, wobei das verzweigte Tetrasaccharid Z der Vorstufen (III), (IV) oder (V) teilweise in geschützter Form, bevorzugt aber in ungeschüzter Form eingesetzt wird.

Vorzugsweise ist X in Verbindung (IV) eine O-Succinimidylgruppe.

Als Lösungsmittel für diese Umsetzung wird vorzugsweise Pyridin oder N,N-Dimethylformamid eingesetzt.

Vorteilhafterweise liegt zur Herstellung einer Verbindung der Formel (I) der Sialinsäurerest im Tetrasaccharidrest Z von Vorstufe (III), (IV) oder (V) in der Lactonform vor.

Zur Herstellung einer Verbindung der Formel (II) ist R² in Vorstufe (VI) geeigneterweise eine Gruppe der Formel (IB), 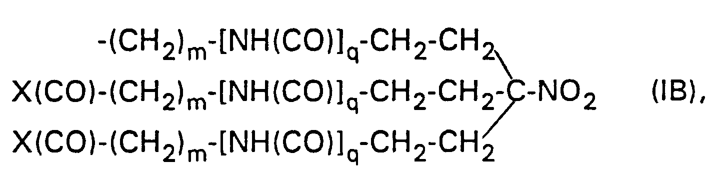 bzw. dient als geeignete Vorstufe eine Verbindung der Formel (VII), 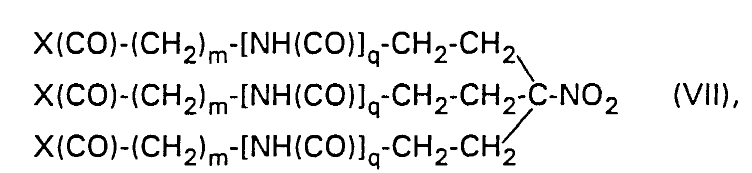 wobei die Variablen m und q die bereits genannten Bedeutungen haben.

Die vorliegende Erfindung betrifft ferner ein Arzneimittel enthaltend eine Verbindung der Formel (I) und gegebenenfalls pharmazeutische Hilfsstoffe.

Die Verbindung der Formel (I) kann insbesondere für die Herstellung eines Arzneimittels zur Vorbeugung oder Heilung von Krankheiten, die durch eine vermehrte Zell-Zell-Adhäsion hervorgerufen werden, verwendet werden.

Die Verbindung (I) eignet sich ferner für die Herstellung eines Mittels zur Diagnose von Krankheiten, die mit einer vermehrten Zell-Zell-Adhäsion einhergehen und zur Herstellung eines synthetischen Vaccinums.

Im folgenden wird die Erfindung detailliert beschrieben, insbesondere in ihren bevorzugten Ausführungsformen. Ferner wird die Erfindung durch den Inhalt der Patentansprüche bestimmt.

### Synthese der Verbindungen (I) mit O-glycosidischer Bindung (Y = O)

Die Verbindungen der Formel (I),

Z-Y-(CH₂)ₙ-[NH(CO)]ₚ-R² I,

in denen der Kohlenhydratligand Z, welcher eine SLeX- oder eine SLeA-Struktur aufweist, O-glycosidisch (d.h. Y = O) und gegebenenfalls über eine aliphatische Spacerkette (CH₂)ₙ mit Aminosäuren, Peptiden oder weiteren lipophilen Resten R² verknüpft wird, lassen sich aus den literaturbekannten Vorstufen der Formel (III) in einfacher Weise durch direkte Ankupplung von den - gegebenenfalls in den Nebenfunktionalitäten geschützten - Reagenzien der Formel (VI) und nachfolgende Abspaltung von Schutzgruppen herstellen.

Beispiele für geeignete Synthesen der Oligosaccharidvorstufen (III) finden sich in der EP 0 601 417 und in Angew. Chem. voraussichtlich Heft 19, 1994 (im Druck), (1994) für Verbindungen mit n = 6 (Hexanolamin-Spacer), in J. Carbohydr. Chem. 1993, 1, 1203 (n = 2 und n = 8) und in J. Carbohydr. Chem. 1994, 13, 641 (Sialyl Lewis-A Typ). Bei den in der vorletzt genannten Publikation aufgeführten Verbindungen kann die Aminofunktion durch Reduktion der Azidvorstufe nach dem Fachmann geläufigen Verfahren eingeführt werden. Demnach ist der Ersatz jeder dritten Methyleneinheit in der Spacerkette (CH₂)ₙ von Verbindungen der Formel (III) durch Sauerstoffatome mit dem Ziel einer besseren Wasserlöslichkeit und Bioverfügbarkeit der angestrebten Endprodukte (I) ebenfalls möglich.

Als Acylkomponenten (VI) kommen aliphatische, cycloaliphatische Carbonsäuren und Aminosäuren sowie Peptide mit bis zu 6 Aminosäuren mit einer freien Säurefunktion (X = OH) infrage, die N-terminal und in den Amino- und Carboxyl-Seitenkettenfunktionen geschützt vorliegen und mit der primären Aminofunktion der Vorstufe (III) unter Bildung einer Amidbindung reagieren können. Hinsichtlich der Auswahl geeigneter Verbindungen (VI) mit X = OH bestehen aufgrund der hohen Reaktivität und guten Zugänglichkeit der Aminofunktion kaum Einschränkungen. Zur Knüpfung der Peptidbindung können die gebräuchlichen Verfahren der Peptidsynthese unter in situ Aktivierung der Säurefunktion, beispielsweise mit Carbodiimiden, angewendet werden. Eine Zusammenstellung aller gebräuchlichen Verfahren findet sich in "Principles of Peptide Synthesis", M. Bodanszky, 2. Auflage 1993, Springer-Verlag.

Aufgrund der Anwesenheit einer freien Säurefunktion und zahlreicher Hydroxylgruppen in den Kohlenhydratbausteinen mit SLeX- und SLeA-Struktur sind diese Methoden wegen möglicher Nebenreaktionen jedoch weniger geeignet. In einer bevorzugten Ausführungsform werden die ungeschützten Kohlenhydratvorstufen (III, IV,V) mit den reinen Aktivestern (VI) zur Reaktion gebracht, um einen eindeutigen Reaktionsverlauf zu gewährleisten.

Beispiele für geeignete Aktivester, die entweder käuflich oder nach den im Lehrbuch über Peptidsynthesen von M. Bodanszky (s.o.) zitierten Verfahren hergestellt werden können, sind Pentafluorphenylester (X = Pentafluorphenoxy), 2,4-Dinitrophenylester (X = 2,4-Dinitrophenoxy) und O-acyliertere, substituierte Hydroxylamine wie beispielsweise N-Hydroxysuccinimidester (X = OSu), die in einer besonders bevorzugten Ausführungsform eingesetzt werden.

Eine besondere Schwierigkeit bei der Durchführung von Reaktionen ungeschützter Kohlenhydratvorstufen wie III (und auch IV und V) besteht in der hohen Hydrophilie der Verbindungen im Vergleich zu den stärker hydrophoben Aktivestern (VI), was einer effizienten Umsetzung der Komponenten in den üblichen organischen Lösemitteln, in wäßrigen Systemen oder in Lösemittelgemischen entgegensteht. In der erfindungsgemäßen Ausführungsform wird die Reaktion bei 0 bis 100°C, bevorzugt bei Temperaturen um Raumtemperatur von 15 bis 35°C in Lösemitteln wie DMF, Dimethylsulfoxid, oder Pyridin, bevorzugt in Pyridin, durchgeführt.

Bevorzugt werden die in Pyridin in geringem Maße, für eine vollständige Umsetzung innerhalb von 1 bis 24 Stunden bei guter Rührung und ausreichender Verdünnung jedoch genügend löslichen, ungeschützten Verbindungen (III) umgesetzt. Besonders bevorzugt werden die lactoiden SLeX - und SLeA-Verbindungen eingesetzt, die formal durch Abspaltung eines Wassermoleküls entstehen können. Diese Verbindungen fallen am Ende der Synthesefolge zu den Vorprodukten nach Abspaltung der Schutzgruppen als Regioisomerengemische an (Beispiel: J. Chem. Soc., Chem. Commun. 1991, 870). Es wurde gefunden, daß diese SLeX/A-Lactone wesentlich besser in organischen Lösemitteln, insbesondere in Pyridin, löslich sind und somit ideale Vorstufen für das erfindungsgemäße Syntheseverfahren darstellen. Aus der Lactongruppe läßt sich dann im letzten Syntheseschritt einfach unter milder basischer Hydrolyse die freie Carboxylgruppe der Sialinsäure freisetzen.

Weitere in den peptidischen Resten R² der Produkte (I) vorhandene Schutzgruppen werden nach den üblichen Verfahren entfernt, beispielsweise Benzylreste in Estern oder Ethern durch katalytische Hydrierung und FMOC-Schutzgruppen von Aminen unter milder basischer Hydrolyse. Die Methoden werden beispielsweise in dem Lehrbuch von Bodanszky (s.o.) und in "Protective Groups in Organic Synthesis" (Th. W. Greene, J. Wiley & Sons, 1981) beschrieben.

Schutzgruppen die unter stark sauren Bedingungen, z.B. Salzsäure oder Trifluoressigsäure abgespalten werden müssen, kommen aufgrund der Säureempfindlichkeit der Verbindungen nicht in Frage. Die Isolierung und Reinigung der Endverbindungen erfolgt durch Entfernung des Lösemittels und mittels chromatographischer Methoden wie HPLC oder FPLC, bevorzugt durch Gelpermeationschromatographie an Trägerphasen wie z.B. Biogel™ oder Chromatographie an Sephadex™ mit Wasser oder Wasser/Alkohol-Gemischen. Die Verbindungen werden durch Dünnschichtchromatographie an Kieselgelen, Elektrospray- oder FAB-Massenspektrometrie, Kernresonanzspektroskopie und biologische Assaysysteme zur Prüfung auf Inhibierung der Zelladhäsion an Selektinrezeptoren charakterisiert.

### Synthese der Verbindungen (I) mit N-glycosidischer Bindung (Y = NHCO)

Die Verbindungen der Formel (I),

Z-Y-(CH₂)ₙ-[NH(CO)]ₚ-R² I,

in denen der Kohlenhydratligand Z, welcher eine SLeX- oder eine SLeA-Struktur aufweist, N-glycosidisch acyliert vorliegt, wobei Y die Amidgruppe NH(CO), p = 0 und n = 0 bedeutet, werden aus dem β-Glycosylamin (IV) durch Acylierung mit einer Verbindung (VI) ganz analog wie oben beschrieben hergestellt. Die Glycosylaminvorstufen (IV) lassen sich prinzipiell aus SLeX oder SLeA durch Ammonolyse der freien Saccharide nach bekannten Verfahren herstellen, beispielsweise durch Umsetzung der Verbindungen (1x) bzw. (1a) mit Ammoniumhydrogencarbonat in Wasser nach dem für freie Saccharide üblichen Verfahren (Glycoconjugate J. 1993, 10, 227 und EP 0 413 675).

In der EP 0 413 675 beispielsweise wird auch die Acylierung der nach diesem Verfahren hergestellten Glycosylamine mit Chloressigsäurederivaten beschrieben, die insbesondere für analytische Anwendungen mit Linkern und fluorophoren Gruppen weiter umgesetzt werden. Die Acylierung erfolgt mit sehr großen Reagenzüberschüssen (5molare Überschüsse) im Mikromaßstab und ist auch wegen der Labilität der freien Glycosylamine im wäßrigen Reaktionsmedium und in Wasser/DMF-Gemischen auf die präparative Synthese mit wertvollen Komponenten der Formeln (IV) und (VI) zu den Produkten (I) bis in den Gramm-Maßstab und darüber hinaus nicht geeignet.

In einer bevorzugten Ausführungsform zur Acylierung der Glycosylamine (IV) werden teilweise geschützte Vorstufen der Oligosaccharide mit SLeA- bzw. SLeX-Struktur eingesetzt, deren Synthese am Beispiel der SLeX-Glycosylaminvorstufe in den Schemata 1 bis 3 gezeigt und in den Beispielen detailliert beschrieben wird: Die selektiv geschützte N-Acetylglucosaminverbindung (3) wird mit dem Thioglycosid-Donor (2) zu (4) fucosyliert. Das Disaccharid (4) wird mit Natriumcyanoborhydrid/Salzsäure in THF (Garegg-Methode) zum Zwischenprodukt (5) reduziert, aus dem nach Galactosylierung mit dem Donor (6) und Entacetylierung nach der Zemplén-Methode das Trisaccharid (7) erhalten wird (Schema 1).

Aus dem Trisaccharid (7) entsteht durch Umsetzung mit Sialinsäure-Donor (8) nach der Thioglycosid-Methode und Umesterung mit Natriummethylat ein Gemisch des Methylesters (10) und des 4'-Lactons (9). Beide Verbindungen können nach dem gleichen Verfahren weiter in die Endprodukte überführt werden. Nach Schema 2 wird das Tetrasaccharid-Lacton (9) mit Raney-Nickel selektiv an der anomeren Azidgruppe zum teilgeschützten Glycosylamin (11) reduziert. Verbindung (11) repräsentiert ein Beispiel für ein teilgeschütztes Vorprodukt der allgemeinen Formel (IV) mit dem Tetrasaccharid-Liganden in der Sialyl Lewis-X Konfiguration Alternativ können zusätzlich die Benzylschutzgruppen mit Palladium/Wasserstoff reduktiv entfernt werden. In beiden Fällen entsteht die β-konfigurierte Aminogruppe in selektiv acylierbarer Form.

Allerdings bietet die erste Variante einer Verwendung des teilgeschützter Glycosylamine in der erfindungsgemäß bevorzugten Ausführungsform den Vorteil, daß für die sich anschließende Acylierung mit den Reaktionspartnern (VI) Verbindungen mit vergleichbarer Hydrophobie in organischen Lösemitteln wie beispielsweise Pyridin, Dichlormethan, Dimethylformamid (DMF), Acetonitril, Tetrahydrofuran (THF) oder Gemische derselben verwendet werden können. Im Beispiel nach Schema 3 wird der Z-geschützte Succinimidester des Tripeptids Gly-Gly-Gly (12) mit dem teilgeschützten Glycosylamin (11) in einem Dimethylformamid/Dichlormethan-Gemisch zum Glycopeptid (13) umgesetzt, aus dem nach reduktiver Entfernung der Schutzgruppen die 4'-lactoide Form (14) eines Endproduktes der allgemeinen Formel (I) erhalten wird. Das Tetrasaccharid-Glycopeptid mit freier Carboxylfunktion an der Sialinsäure, im Beispiel die Endverbindung (I-1), wird durch milde Hydrolyse des Lactonringes mit verdünnten wäßrigen Basen, wie zum Beispiel mit 0.1 bis 0.01 N Natronlauge, und anschließendes Ansäuern erhalten.

Die Verbindungen (I) mit der Sialyl Lewis-A Konfiguration werden ganz analog wie oben beschrieben erhalten: Die Verbindungen dieses Strukturtyps entstehen durch Vertauschung der Glycosylierungskomponenten bei der Anhängung der Seitenarme in den 3- und 4-Positionen des N-Acetylglucosamin-Bausteins. So wird in Analogie zu den Schemata 1 bis 2 erst in der freien 3-Position galactosyliert und dann nach reduktiver Öffnung des Benzylidenacetals in der freien 4-Position fucosyliert. Die weitere Synthese verläuft ganz analog dem für (I-1) beschriebenen Verfahren.

Der Ankupplung von Verbindungen der Formel (VI) an Glycosylamine (IV) über Succinimid-Aktivester (I; X = OSu) ist auch hier die bevorzugte Ausführungsform der Erfindung. Einzige Nebenkomponente dieser Reaktion ist das zwangsläufig anfallende N-Hydroxysuccinimid. Da sonst keine weiteren Reagenzien oder Aktivatoren benötigt werden, können die Endprodukte durch einfache Filtration über stationäre Chromatographie-Phasen wie zum Beispiel Biogel® mit Wasser als Laufmittel oder an Sephadex® bzw. typähnlichen Materialien mittels Wasser oder Gemischen von Wasser mit organischen Lösemitteln als Eluenten gereinigt werden. Die Reaktionspartner der Formeln (IV) und (VI) werden stöchiometrisch oder in Überschüssen von 1 bis 10 Mol% der jeweils in größerer Menge verfügbaren Komponente eingesetzt. Die jeweilige Überschußkomponente wird nach genannten Chromatographieverfahren abgetrennt.

Die Succinimidester (VI) können in isolierter Form (X = OSu) oder in situ aus den Carbonsäuren (VI, X = OH) mit dem käuflichen Reagenz TSTU [O-(N-Succinimidyl)-N,N,N',N'-tetramethyl-uronium-terafluoroborat] oder nach anderen geläufigen Verfahren der Peptidsynthese (s. zit. Lit.: M. Bodanszky) erhalten werden. So können auch HOBT-Ester (HOBT = 1-Hydroxybenzotriazolyl), die in situ aus Carbonsäuren hergestellt werden, oder andere Spezialverfahren, die spezielle zur Acylierung chemisch labiler Glycosylamine entwickelt wurden, eingesetzt werden. Ein Beispiel für letztere ist die Verwendung von 3-Acyl-5-methyl-1,3,4-thiadiazol-2(3H)-thionen (J. Carbohydr. Chem. 1994, 13, 737).

Beim erfindungsgemäß bevorzugten Succinimidesterverfahren zur Synthese der Produkte (I) und (II) können im Gegensatz zu den literaturbekannten Verfahren, beispielsweise dem HBTU-Verfahren (Glycoconjugate J. 1993, 10, 227) zur Acylierung freier Glycosylamine, die Tetrasaccharidvorstufen (III), (IV) und (V) auch in der freien Sialinsäure-Form verwendet werden. Bevorzugt werden jedoch die in der Lactonform vorliegenden Verbindungen eingesetzt.

Die im US-Patent 5,280,113 beschriebenen und beanspruchten Methoden zur Anknüpfung von Peptiden, die aus 5 bis 25 Aminosäuren bestehen, an ungeschützte Glycosylamine sind nicht auf Sialinsäure enthaltende Glycosylamine (IV) anwendbar. So würde die Aktivierung einer Carbonsäure (VI. X = OH) beispielsweise mittels HBTU/HOBT zwangsläufig auch zu einer Aktivierung der Sialinsäure und zu somit zu einem anderen Reaktionsverlauf führen.

Die Charakterisierung der Produkte erfolgt in der gleichen Weise wie oben für die O-Glycoside der allgemeinen Formel (I) aufgeführt.

Die Verbindungen der Formel (I), in denen der Ligand mit SLeX- bzw. mit SLeA-Struktur gleichfalls N-glycosidisch acyliert vorliegt (Y = NHCO), jedoch p = 1 und (CH₂)ₙ eine aliphatische Spacerkette mit n = 1 bis 10 C-Atomen bedeuten, werden durch Acylierung von entsprechenden Vorstufen der allgemeinen Formel (V) völlig analog wie die aus Vorstufen (III) herstellbaren Verbindungen (I) (s. oben) hergestellt.

Die Vorstufen der allgemeinen Formel (V) werden ihrerseits aus den Vorstufen (IV) mit den entsprechenden, oben beschriebenen Verfahren, durch Acylierung mit endständigen, N-terminal geschützten Aminocarbonsäuren bzw. deren Aktivestern hergestellt. Beispiele für geeignete endständige Aminocarbonsäuren sind Glycin (n = 1) und 6-Aminocapronsäure (n = 6). Geeignete Schutzgruppen für die N-terminale Aminogruppe sind die hydrogenolytisch abspaltbaren Azidound Benzyloxycarbonyl- (Z-) Schutzgruppen sowie die unter milden basischen Bedingungen abspaltbaren Trifluoracetyl- und Fluorenylmethoxycarbonyl-Schutzgruppen (FMOC).

So erhält man beispielsweise in Analogie zu der in Schema 3 beschriebenen Synthesesequenz bei Verwendung von SuO(CO)(CH₂)₆NH-Z nach Abspaltung der Z-Schutzgruppe die Verbindung (V) mit n = 6.

Das erfindungsgemäße Verfahren zur Synthese der Kohlenhydratkonjugate (I) hat in seiner bevorzugten Ausführungsform, der Umsetzung von aminofunktionalisierten Kohlenhydratvorstufen (III, IV oder V) mit den Verbindungen (VI, X = OSu) den Vorteil, daß eine große Vielfalt von Carbonsäuren, Aminosäuren und Peptiden mit freier Carbonsäurefunktion, welche sämtlich in ihre OSu-Ester überführbar sind, verwendbar sind.

In den Schemata 4/1 bis 4/3 sind Beispiele für so erhältliche Verbindungen aufgeführt, die zur Illustration der Erfindung und der durch die vielfältige Variierbarkeit von Rest R² gegebenen Anwendungen dienen sollen. Die Verbindungen (I) zeichnen sich vor allem auch durch ihre verstärkte Affinität zu Selektinrezeptoren aus.

Eine weitere Variante des erfindungsgemäßen Verfahrens zur Synthese von neuen lipophilen Oligosaccharidliganden aus den aminofunktionalisierten Kohlenhydratvorstufen (III), (IV) oder (V) besteht in der Addition geeigneter Isocyanate oder Isothiocyanate als reaktive Carboxylkomponenten alternativ zu den Aktivestern der Formel (VI). Dabei entstehen Produkte der Formel (I) mit (Thio)harnstoffbindungen NH(CO)NH bzw. NH(CS)NH anstelle von Amidbindungen, was kaum Unterschiede in der Affinität der Verbindungen zu den Selektinrezeptoren hervorruft. Ein Beispiel hierfür ist die in Schema 4/3 angeführte Synthese von (I-16) durch Umsetzung einer Vorstufe der Formel (III-1) mit dem käuflichem Reagenz Fluorescein-isothiocyanat in Pyridin als Lösungsmittel.

Die im Gramm-Maßstab leicht herstellbare fluorophore Verbindung (I-16) eignet sich ebenso wie die ganz analog aus der entsprechenden Vorstufe mit der Sialyl Lewis-A Konfiguration erhältliche Verbindung besonders gut für in vitro- und in vivo-Studien der Zelladhäsion. Diese Verbindungen sind durch ihre optimale Spacerlänge besser geeignet als die käuflichen Verbindungen der Firma Oxford Glycosystems (Katalog-Nr. F-02026 und F-02028), in denen der fluorophore Farbstoff über den starren, kurzen Glycylspacer an die Oligosaccharide geknüpft wurde. Der flexiblere Spacer in (I-16) präsentiert den Kohlenhydratliganden frei von störenden Einflüssen des relativ großen Farbstoffmoleküls. Dies zeigen die im Vergleich zum freiem Sialyl Lewis-X-Tetrasaccharid (IC₅₀-Werte 1 bis 2 mM) in (I-16) noch deutlich verbesserten IC₅₀-Daten (Tabelle 1).

Das Glycokonjugat (I-2) enthält die antiadhäsive Peptidsequenz Arg-Gly-Asp-Ala, die zusätzlich als Bindungsstelle für Integrine, beispielsweise für das Glycoprotein GP IIb/IIIa, fungieren kann. Das Peptid Arg-Gly-Asp-Ala allein bindet nicht an Selektine.

Das Biotin-Glycokonjugat (I-11) bildet Additionsverbindungen mit Avidin und mit Streptavidin, in denen bis zu 4 Sialyl Lewis-X Liganden gebunden vorliegen.

Das Glycokonjugat (1-12) enthält das Strukturelement des chemotaktischen Peptids N-Formyl-Met-Leu-Phe (fMLP), von dem ein synergistischer Einfluß bezüglich eines reduzierten Rolling von Neutrophilen in vivo zu erwarten ist, (Blood 1993, 82, 1165-1174).

Die Glycokonjugate (1-13) und (I-14) enthalten das Strukturelement von Lipopeptid-Vaccinen des Pam₃Cys(Ser)-Typs. Lipopeptide mit diesem Strukturelement bewirken im Gegensatz zu Peptiden eine primäre Immunantwort auf einen Antigenreiz, indem sie rasch durch Zellmembranen transportiert und dann im Cytoplasma internalisiert werden. Dieser Prozess führt zur Aktivierung von Makrophagen und zu einer Antigen-spezifischen Immunantwort.

Die durch T-Zellen vermittelte Immunantwort gegen Proteine ist ein in der Tumorforschung bekannter Mechanismus, während hier über die Rolle von Kohlenhydraten noch keine Erkenntnisse vorliegen. Voraussetzung hierfür ist die Präsentation von Peptiden, die an MHC-Proteine binden. In der WO 93/21948 werden immunogene Konjugate zur Erzeugung einer von T-Zellen vermittelten Immunität gegen Tumor-assoziierte Kohlenhydratantigene vorgeschlagen, die MHC-1 bindende Peptide und einen immunogenen Kohlenhydratliganden enthalten.

Dagegen ist von den Verbindungen (I-13) und (I-14) als potentiellen synthetischen Vaccinen eine spezifische Immunantwort auf das Sialyl Lewis-X-Antigen zu erwarten, die auch unabhängig vom MHC-Mechanismus ausgelöst werden kann. Entsprechendes gilt für die in analoger Weise erhältliche Verbindung mit Sialyl Lewis-A-Ligandenstruktur. Die Immunantwort könnte bei entzündlichen Erkrankungen und Autoimmunerkrankungen ohne Hilfe makromolekularer Carrier oder Adjuvantien indirekt zu einer verminderten Leukozyten-Adhäsion und -Infiltration beitragen, indem die Zahl verfügbarer Antigenliganden reduziert wird.

Dieses Konzept stellt eine Erweiterung der in der Antitumortherapie bekannten Strategie eines Einsatzes synthetischer, niedermolekularer Lipopeptid-Tumorantigen-Konjugate dar, über das erstmals in J. Am. Chem. Soc. 1994, 116, S. 395 berichtet wurde. Im Vergleich zu den dort verwendeten Glycokonjugaten vom Tn-Antigen-Typ sind die erfindungsgemäßen Verbindungen (I-13) und (1-14) aufgrund ihrer größeren Kohlenhydratstrukturen besser zur Auslösung einer Immunantwort geeignet. Eine Verstärkung der Immunantwort unter Beteiligung von T-Zellen kann ebenfalls erwartet werden.

### Synthese der Verbindungen (II)

Die Verbindungen der Formel (I),

Z-Y-(CH₂)ₙ-[NH(CO)]ₚ-R² I,

bei welchen R² eine Gruppe der Formel (IA) darstellt, 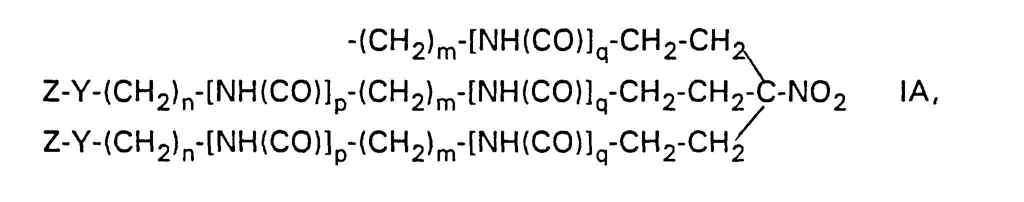 werden im folgenden Verbindungen der Formel (II) genannt,

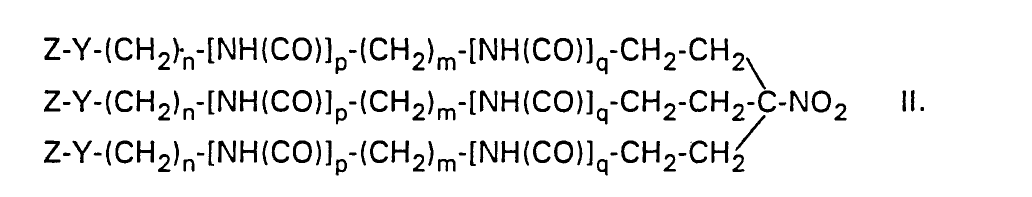

Die Verbindungen (II), in denen drei Kohlenhydratliganden Z, die eine SLeX- oder SleA-Struktur aufweisen, N-glycosidisch oder O-glycosidisch acyliert und gegebenenfalls über die aliphatischen Spacerketten (CH₂)ₙ und (CH₂)ₘ verlängert über Amidbindungen an die Gerüstverbindung 4-(2-Carboxyethyl)-4-nitroheptandisäure (VII-1, X = OH, m = q = 0) angeknüpft vorliegen, werden durch dreifache Umsetzung jeweils einer Verbindung der Formel (III), (IV) oder (V) mit der Verbindung (VII-1) oder mit einem kettenverlängerten Derivat der allgemeinen Formel (VII) 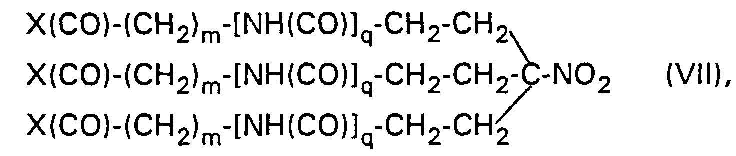 hergestellt.

Grundsätzlich können auch hier die zur Synthese von Verbindungen (I) mit monovalenter Kohlenhydratstruktur eingesetzten Methoden zur Knüpfung von Amidbindungen angewandt werden. Bedingt durch die trivalente Natur einer Verbindung der Formel (VII) werden hierbei aber kaum trennbare Gemische mono-, di- und trivalenter Produkte erhalten. In der bevorzugten Ausführungsform der Erfindung, in der leicht Gramm-Mengen definiert trivalenter Produkte (II) hergestellbar sind, werden die neuen (noch nicht beschriebenen) Tris-OSu-Ester von (VII, X = OSu) verwendet, die aus der käuflichen Tricarbonsäure (VII-1) wie in den folgenden Beispielen erläutert synthetisiert werden (Su = Succinimidylrest):

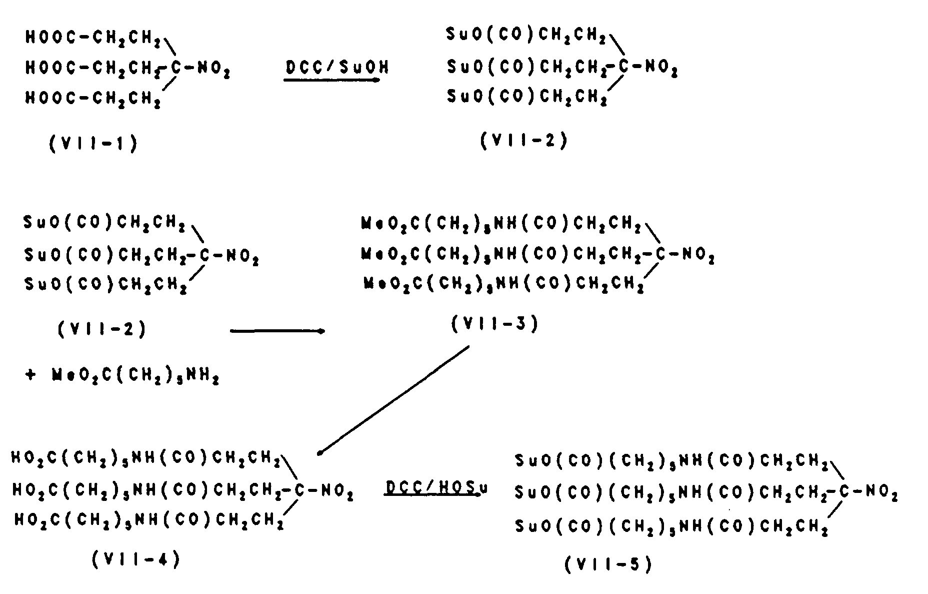

Beispiele für so hergestellte Verbindungen (II) finden sich in Schema 5. Beispielsweise wird die Verbindung (II-1) durch Umsetzung von (VII-2) mit mindestens drei Äquivalenten einer Verbindung (IV) mit der SLeX-Konfiguration erhalten. Die Verbindung (II-2) entsteht in analoger Weise aus einer Vorstufe (III), in der n = 6 bedeutet. Die Verbindung (II-3) wird beispielsweise aus (VII-5) und der Verbindung (IV) mit SLeX-Konfiguration erhalten.

Von einer Verbindung (III), (IV) oder (V) abgeleitete, trivalente Produkte lassen sich entweder durch Anknüpfung von Spacerketten, beispielsweise an die Vorstufe (VII-1), und anschließende Kupplung mit (III), (IV) oder (V) wie dargelegt herstellen oder alternativ durch Vertauschung der Synthesefolge, indem die an der Aminogruppe geschützte Aminosäure zuerst an (III), (IV) oder (V) gekuppelt wird und das Zwischenprodukt nach Entschützen der Aminogruppe dann dreifach an den Tris-OSu-Ester einer Gerüstverbindung (VII) angehängt wird.

Die erste Alternative ist zu bevorzugen, weil die wertvollere Verbindung (III), (IV) oder (V) erst im letzten Schritt eingesetzt wird.

Die Durchführung der Synthese einer Verbindung (II), die Reinigung der Produkte und deren Charakterisierung erfolgen ganz analog wie für die monovalenten Verbindungen (I) beschrieben.
Pro Reaktivgruppe von (VII) wird die Verbindung (III), (IV) oder (V) in maximal 1 bis 10 mol% Überschuß eingesetzt.

Die in den Verbindungen (II) vorhandene Nitrogruppe läßt sich nach dem Fachmann geläufigen Verfahren, beispielsweise mit Raney-Nickel und Wasserstoff, zur Aminogruppe reduzieren. Die gleichen Endverbindungen mit einer Aminogruppe wären grundsätzlich auch ausgehend von der käuflichen Verbindung 4-(2-Carboxyethyl)-4-amino-heptandisäure herstellbar. Hierfür müßte allerdings der Umweg über die Einführung einer geeigneten Aminoschutzgruppe gewählt werden, wobei nur basisch oder hydrogenolytisch abspaltbare Aminoschutzgruppen infrage kommen. Eine acidolytisch abspaltbare Boc-Schutzgruppe würde keinesfalls zu den der Formel (II) entsprechenden Produkten (mit einer Aminogruppe anstelle der Nitrogruppe) führen, weil unter diesen Bedingungen die Fucose ebenfalls entfernt würde.

Das Synthesekonzept für polyvalente Verbindungen vom Typ der Verbindungen (II) ist nicht auf trivalente Glycokonjugate beschränkt. Höhervalente Glycoconjugate, beispielsweise tetravelente Verbindungen, können in analoger Weise aus den entsprechenden Oligocarbonsäuren, beispielsweise aus der 2,2'-Bis(2-carboxyethyl)-pentandisäure, und zwar ebenfalls bevorzugt über deren N-Hydroxysuccinimidester, hergestellt werden.

Eigenschaften der erfindungsgemäßen Verbindungen (I) bzw. (II) als Inhibitoren der Zelladhäsion sowie ihre therapeutische und diagnostische Verwendung.

Die Verbindungen der Formeln (I) bzw. (II) eignen sich als pharmakologisch wirksame Stoffe, insbesondere als Wirkstoffe zur Vorbeugung oder Heilung von Krankheiten, die durch eine vermehrte Zell-Zell-Adhäsion hervorgerufen werden. Die erfindungsgemäßen Verbindungen zeigen insbesondere eine verbesserte Wirksamkeit der Inhibierung der durch Selektinrezeptoren vermittelten Zelladhäsion.

Die Kohlenhydratkonjugate dürfen bei Applikationen in vivo keine nachteiligen Nebenwirkungen aufweisen. So sind im Falle intravenöser Anwendungen beispielsweise hämolytische und unerwünschte immunogene Eigenschaften zu vermeiden. Die Enzyme der Blutgerinnungskaskade dürfen nicht aktiviert werden, um die Bildung von Thromben auszuschließen.

Die erfindungsgemäßen Kohlenhydratkonjugate und ihre physiologisch verträglichen Salze eignen sich aufgrund ihrer wertvollen pharmakologischen Eigenschaften sehr gut zur Anwendung als Heilmittel bei Säugern, insbesondere dem Menschen. Die Arzneimittel eignen sich vorzugsweise zur Prophylaxe und/oder Therapie von Krankheiten, die unter Beteiligung entzündlicher Prozesse ablaufen, vorzugsweise von Myokardinfarkt und Ischämie, Nachinfarktsyndrom, Schocklunge des Erwachsenen, Septischer Schock, Schlaganfall, akute und chronische Organabstoßung, Vasculitis, entzündliche Erkrankungen der Haut, rheumatische Arthritis, Restenosis nach Angioplastie sowie metastasierender Tumore.

Die erfindungsgemäßen Arzneimittel werden im allgemeinen intravenös, oral oder parenteral oder als Implantate verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierte Wirkstoff-Freigabe, bei deren Herstellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische und pflanzliche Öle, Polyethylenglykole und Lösungsmittel, wie etwa steriles Wasser, Alkohole, Glycerin und mehrwertige Alkohole genannt. Vorzugsweise werden die pharmazeutischen Präparate in Dosierungseinheiten hergestellt und verabreicht. Bei festen Dosierungseinheiten sind Tabletten, Kapseln und Suppositorien bevorzugt.

Für die Behandlung eines Patienten sind je nach Wirksamkeit der Verbindung, Art der Applikation, Art und Schwere der Erkrankung, Alter und Körpergewicht des Patienten, unterschiedliche Tagesdosen notwendig. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleiner Dosierungseinheiten als auch durch Mehrfachgabe unterteilten Dosen in bestimmten Intervallen erfolgen. Die zu verabreichende Tagesdosis kann außerdem von der Anzahl der während des Krankheitsverlaufs exprimierten Rezeptoren abhängig sein. Es ist vorstellbar, daß im Anfangsstadium der Krankheit nur wenige Rezeptoren auf der Zelloberfläche expirmiert werden und demzufolge die zu verabreichende Tagesdosis geringer ist als bei stark erkrankten Patienten.

Die erfindungsgemäßen Arzneimittel werden dadurch hergestellt, daß man das Kohlenhydratkonjugat mit üblichen Träger- sowie gegebenenfalls Zusatz- und/oder Hilfsstoffen in die bzw. eine geeignete Darreichungsform bringt.

Primärassays zur Untersuchung der Wirkung von Kohlenhydratkonjugaten der Formeln (I) bzw. (II) auf die Zellanheftung an rekombinante, lösliche Selektin-Fusionsproteine.

Die Durchführung dieser Assays zur Messung der inhibierenden Wirksamkeit der erfindungsgemäßen Verbindungen auf die Zellanheftung von promyelozytischen Zellen mittels Selektinen wird ausführlich in der europäischen Veröffentlichungsschrift EP 0 601 417 beschrieben.

Primärassays zur Untersuchung der Wirkung von Kohlenhydratkonjugaten auf die Zellanheftung an rekombinante lösliche Selektin-Fusionsproteine Um die Wirksamkeit der Kohlenhydratkonjugate auf die Interaktion zwischen den E- und P-Selektinen (alte Nomeklatur ELAM-1 bzw. GMP-140) mit ihren Liganden zu testen, wird ein Assay verwendet, der jeweils nur für eine dieser Interaktionen spezifisch ist. Die Liganden werden in ihrer natürlichen Form als Oberflächenstrukturen auf promyelozytischen HL60 Zellen angeboten. Da HL60 Zellen Liganden und Adhäsionsmoleküle unterschiedlichster Spezifität aufweisen, kann die gewünschte Spezifität des Assays nur über den Bindungspartner erbracht werden. Als Bindungspartner wurden gentechnisch hergestellte lösliche Fusionsproteine aus der jeweils extrazytoplasmatischen Domäne von E- bzw. P-Selektin und der konstanten Region eines humanen Immunglobulins der Subklasse IgG1 verwendet.

### Herstellung von L-Selektin-IGG1

Zur Herstellung von löslichem L-Selektion-IgG1 Fusionsprotein wurde das von Walz et al., 1990 publizierte genetische Konstrukt "ELAM-Rg" verwendet.

Zur Expression wurde die Plasmid DNA in COS-7 Zellen (ATCC) mittels DEAE-Dextran transfiziert (Molekularbiologische Methoden: siehe Ausubel, F. M., Brent, R., Kingston, R. E., Moore, D. D., Seidman, J. G., Struhl, K. und Smith, J. A. 1990. Current Protocols in Molecular Biology, John Wiley, New York). Sieben Tage nach der Transfection wird der Kulturüberstand gewonnen, durch Zentrifugation von Zellen und Zellfragmenten befreit und auf 25 mM Hepes pH 7,0, 0,3 mM PMSF, 0,02 % Natriumazid gebracht und bei +4°C aufgehoben.

Walz, G., Aruffo, A., Kolanus, W., Bevilacqua, M. und Seed, B. 1990. Recognition by ELAM-1 of the sialyl-Lex determinant on myeloid and tumor cells. Science 250, 1132-1135.

### Herstellung von P-Selektin-IGG1

Zur Herstellung des löslichen P-Selektin-IgG1 Fusionsproteins wird das von Aruffo et al., 1991 publizierte genetische Konstrukt "CD62Rg" verwendet. Die weitere Vorgehensweise entspricht der unter A1 dargestellten Herstellung von L-Selektin-IgG1.

Aruffo, A., Kolanus, W., Walz, G., Fredman, P. und Seed, B. 1991. CD62/-P-Selectin recognition of myeloid and tumor cell sulfatides. Cell 67, 35-44.

### Herstellung von CD4-IGG1

Zur Herstellung des löslichen CD4-IgG1 Fusionsproteins wird das von Zettlemeissl et al., 1990 publizierte genetische Konstrukt "CD4:IgG1 hinge" verwendet. Die weitere Vorgehensweise entspricht der unter A1 dargestellten Herstellung von L-Selektin-IgG1.

Zettelmeissl, G., Gregersen, J.-P., Duport, J. M., Mehdi, S., Reiner, G. und Seed, B. 1990. Expression and characterization of human CD4: Immunoglobulin Fusion Proteins. DNA and Cell Biology 9, 347-353.

Durchführung des HL60 Zelladhäsionsassays auf rekombinanten, löslichen Adhäsionsmolekülen
1. 96er Mikrotitertestplatten (Nunc Maxisorb) werden mit 100 µl eines in 50 mM Tris pH 9,5 verdünnten (1 + 100) Ziege anti human IgG Antikörpers (Sigma) 2 Stunden bei Raumtemperatur inkubiert. Nach Entfernen der Antikörperlösung wird einmal mit PBS gewaschen.
2. 150 µl des Blockierungspuffers werden für 1 Stunde bei Raumtemperatur in den Näpfchen belassen. Die Zusammensetzung des Blockierungspuffers ist: 0,1 % Gelatine, 1 % BSA, 5 % Kalbserum, 0,2 mM PMSF, 0,02 % Natriumazid. Nach Entfernen des Blockierungspuffers wird einmal mit PBS gewaschen.
3. In die Näpfchen werden je 100 µl Zellkulturüberstand von entsprechend transfektierten und exprimierenden COS-Zellen pipettiert. Die Inkubation erfolgt 2 Stunden bei Raumtemperatur. Nach Entfernen des Zellkulturüberstandes wird einmal mit PBS gewaschen.
4. In die Näpfchen werden 20 µl Bindungspuffer gegeben. Der Bindungspuffer hat die Zusammensetzung: 50 mM Hepes, pH 7,5; 100 mM NaCI; 1 mg/ml BSA; 2 mM MgCl₂; 1mM CaCl₂; 3 mM MnCl₂; 0,02-% Natriumazid; 0,2 mM PMSF. Dazu werden 5 µl der Testsubstanz pipettiert, durch Schwenken der Platte vermischt und 10 Minunten bei Raumtempeartur inkubiert.
5. 50 ml einer HL60 Zellkultur mit 200.000 Zellen/ml werden 4 Minunten bei 350 g zentrifugiert. Das Pellet wird in 10 ml RPMI 1640 resuspendiert und die Zellen erneut zentrifugiert. Zur Markierung der Zellen werden 50 µg BCECF-AM (Molecular Probes) in 5 µl wasserfreiem DMSO aufgelöst; anschließend werden 1,5 ml RPMI 1640 auf die BCECF-AM/DMSO-Lösung gegeben. Mit dieser Lösung werden die Zellen resuspendiert und 30 Minuten bei 37°C inkubiert. Nach zweiminütiger Zentrifugation bei 350 g wird das markierte Zellpellet in 11 ml Bindungspuffer resuspendiert und die resuspendierten Zellen in 100 µl Aliquots in die Mikrotiterplatten-Näpfchen verteilt. Die Platte wird 10 Minunten bei Raumtemperatur stehen gelassen, um die Zellen auf den Boden der Testplatte sedimentieren zu lassen. Dabei haben die Zellen Gelegenheit an das beschichtete Plastik zu adhärieren.
6. Zum Abstoppen des Tests wird die Mikrotiterplatte im 45° Winkel gänzlich in den Stoppuffer getaucht (25 mM Tris, pH 7,5; 125 mM NaCI; 0,1 % BSA; 2 mM MgCl₂; 1 mM CaCl₂; 3 mM MnCl₂; 0,02 % Natriumazid). Durch Invertierung wird der Stoppuffer aus den Näpfchen entfernt und die Prozedur noch zweimal wiederholt.
7. Die Messung der in den Näpfchen festhaftenden, BCECF-AM-markierten Zellen erfolgt in einem Cytofluorimeter (Millipore), bei einer Empfindlichkeitseinstellung von 4, einer Anregungswellenlänge von 485/22 nm und einer Emissionswellenlänge von 530/25 nm.

Die entsprechend erhaltenenen Ergebnisse zur Bestimmung der HL60-Zelladhäsion an die rekombinanten, löslichen Adhäsionsmoleküle E-Selektin und P-Selektin finden sich in Tabelle 1.

Weitere geeignete Assaysysteme, wie beispielsweise die Untersuchung der Zellanheftung an stimulierte, humane Endothelzellen (HUVEC), an Gefrierschnitte von lymphatischem Gewebe und auf die Leukozytenadhäsion in der Ratte in vivo mittels Intravitalmikroskopie, werden in der zitierten Veröffentlichung gleichfalls ausführlich beschrieben.

Überraschenderweise zeigen vor allem die durch Anhängen von relativ großen lipohilen Resten, Aminosäuren, Lipopeptiden und Peptiden an die Tetrasaccharide mit Sialyl Lewis-X bzw. Sialyl Lewis-A Struktur erhältlichen Verbindungen eine deutlich verbesserte Wirksamkeit (Daten siehe Tabelle 1).

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht werden.

### Beispiel 1:

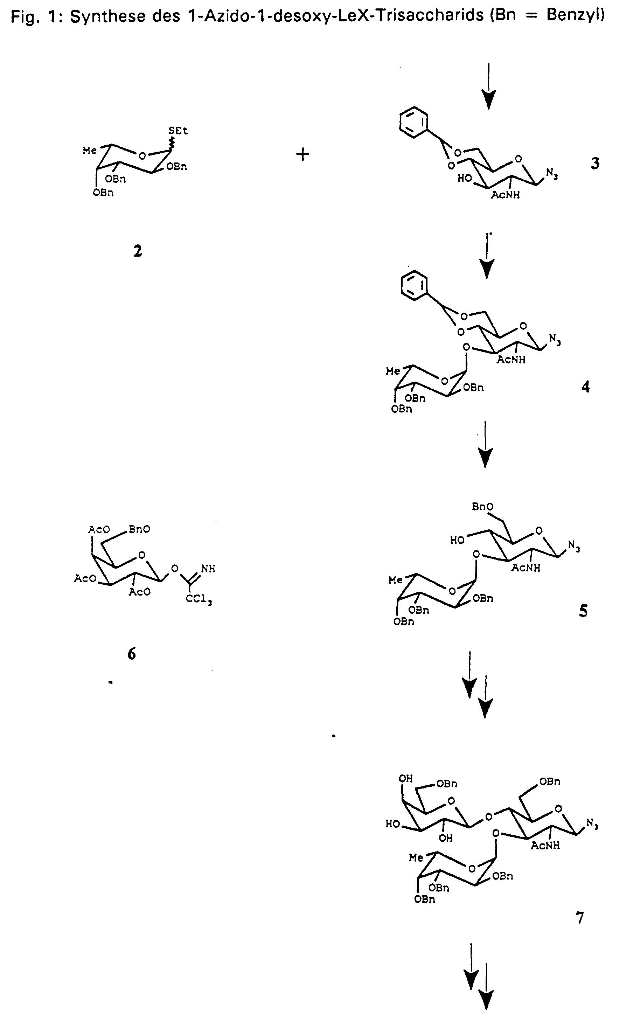

### Synthese von (2,3,4-Tri-O-benzyl-α-L-fucopyranosyl)-(1→3)-2-acetamido-1-azido-4,6-O-benzyliden-1,2-didesoxy-β-D-glucopyranose (4) (Fig. 1):

2-Acetamido-1-azido-4,6-O-benzyliden-1,2-didesoxy-β-D-glucopyranose (3) (36.0 g, 108 mmol) undEthyl-2,3,4-tri-O-benzyl-1-thio-β-L-fucopyranosid (2) (67.2 g, 140 mmol) werden 1 Stunde in Dichlormethan/DMF (700 ml, 1:1) über Molekularsieb (3A) gerührt. Nach Zugabe von Tetra-butylammoniumbromid (62.7 g, 194 mmol) und Kupfer(II) bromid (38.6 g, 173 mmol) wird 16 Stunden bei Raumtemperatur gerührt. Man filtriert durch Kieselgel, spült mit Dichlormethan (1.5 L), wäscht mit gesättigter Natriumhydrogencarbonatlösung gefolgt von gesättigter Natriumchloridlösung. Entfernen der Lösungsmittel im Vakuum und Säulenchromatographie an Kieselgel (i-Hexan/Essigsäureethylester 4:1) liefert das Disaccharid (4) (68.0 g, 91 %).
[α]_{D}²⁰ = -143.3° (1 / CH₂Cl₂); ¹H-NMR (300 MHz, CDCl₃): δ = 0.88 (d, 3H, 6-H_{Fuc}), 1.61 (s, 3H, NHAc), 5.05 (d, 1H, 1-H_{Fuc}), 5.52 (s, 1H, CH-Benzyliden), 7.25-7.4 (m, 20H, Benzyl).

### Beispiel 2:

### Synthese von (2,3,4-Tri-O-benzyl-α-L-fucopyranosyl)-(1→3)-2-acetamido-1-azido-6-O-benzyl-1,2-didesoxy-β-D-glucopyranose(5) (Fig. 1):

Verbindung (4) (69.0 g, 100 mmol) und Natriumcyanoborhydrid (63.0 g, 1.0 mol) werden in Tetrahydrofuran (1 l) über Molekularsieb (120 g, 4 Å) 1 Stunde bei Raumtemperatur gerührt. Unter Kontrolle mittels Dünnschichtchromatographie (DC) tropft man bis zum Abklingen der anfangs heftigen Gasentwicklung vorsichtig eine Lösung von HCI in Ether zu, wobei ein farbloser Niederschlag ausfällt. Es wird zusätzliches Natriumcyanoborhydrid und weitere Lösung von HCI in Ether unter sorgfältiger DC-Kontrolle eingetragen, bis ein fast vollständiger Umsatz erreicht ist. Man neutralisiert mit Natriumhydrogencarbonat, nimmt mit Dichlormethan auf, wäscht mit gesättigter Natriumhydrogencarbonatlösung, Ethanolamin (5 % in Wasser) gefolgt von gesättigter Natriumchloridlösung. Einengen und Säulenchromatographie an Kieselgel (Toluol/Essigsäureethylester 4:1) liefert das Disaccharid (5) (53.0 g, 76 %) als amorphen Feststoff.
[α]_{D}²⁰ = -83,9° (1 / CH₂Cl₂); ¹H-NMR (300 MHz, CDCl₃): δ= 1.16 (d, 3H, 6-H_{Fuc}), 1.58 (s, 3H, NAc), 4.95 (d, 1H, 1-H_{Fuc}), 7.1-7.6 (m, 20H, Benzyl).

### Beispiel 3:

### Synthese von (6-O-Benzyl-β-D-galactopyranosyl)-(1→4)-[(2,3,4-tri-O-benzyl-α-Lfucopyranosyl)(1→3)]-(1→3)-2-acetamido-1-azido-6-O-benzyl-1,2-didesoxy-β-Dglucopyranose (7) (Fig. 1):

Eine Lösung von Verbindung (5) (27.0 g, 35.9 mmol) und O-(2,3,4-Tri-O-acetyl-6-O-benzyl-α/β-galactopyranosyl)-trichloracetimidat (6) (30.8 g, 53.8 mmol) in 500 ml Dichlormethan wird 1h über Molekularsieb (4A) gerührt. Zu der Lösung tropft man binnen 3 Stunden TMSOTf (797 mg, 3.6 mmol) und neutralisiert anschließend mit festem Natriumhydrogencarbonat. Das Rohprodukt wird mit Dichlormethan (1.5 L) versetzt und mit gesättigter Natriumhydrogencarbonatlösung gefolgt von gesättigter Natriumchloridlösung gewaschen. Nach Trocknen mit MgSO₄ und Einengen im Vakuum wird das Rohprodukt in Methanol (1 l) gelöst und mit 10 ml einer 0.1 M Lösung von NaOCH₃ in Methanol versetzt. Nach 2 Stunden Rühren bei Raumtemperatur wird mit lonenaustauscher (Amberlite® IR-120) neutralisiert und das Lösemittel im Vakuum entfernt. Anschließende Säulenchromatographie (SC) an Kieselgel (Dichlormethan/Methanol 40:1) liefert Verbindung (7) (16.5 g, 46 %) als amorphen Feststoff.
[α]_{D}²⁰ = -88,3 (1 / CH₂Cl₂); ¹H-NMR (300 MHz, CDCl₃): δ = 1.14 (d, 3H, 6-H_{Fuc}), 1.62 (s, 3H, NHAc), 5.14 (d, 1H, 1-H_{Fuc}), 6.12 (d, 1H, NHAc), 7.1-7.5 (m, 25H, Benzyl).

### Beispiel 4:

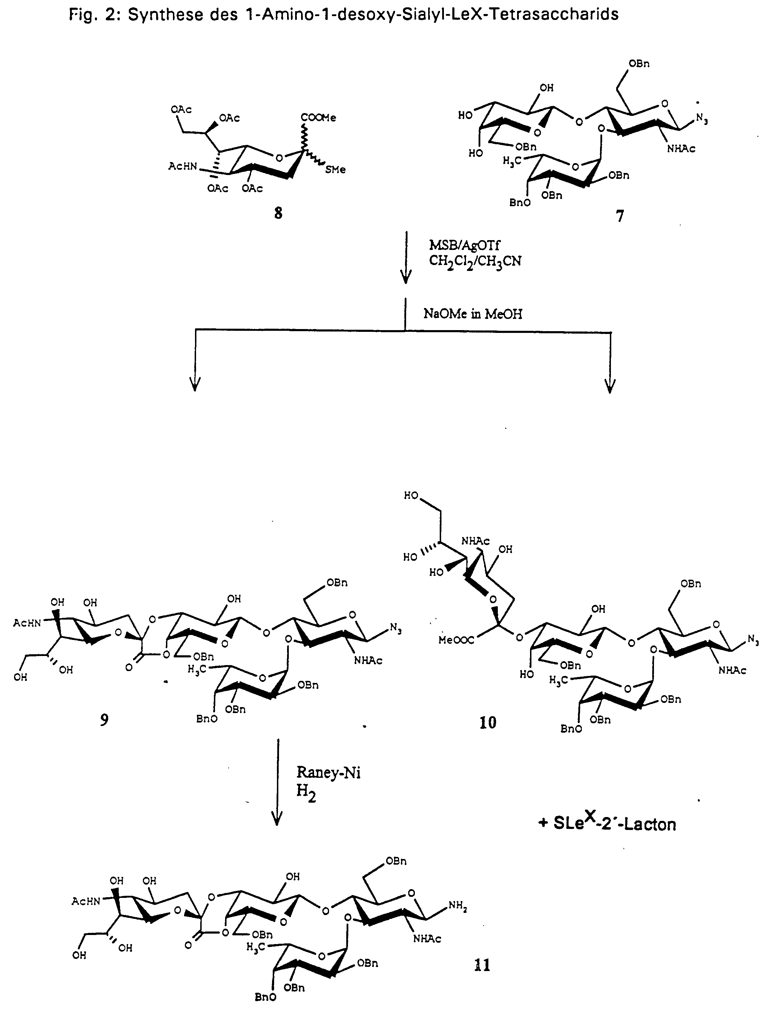

### Synthese von (5-Acetamido-3,5-didesoxy-D-glycero-α-D-galacto-2-nonulopyranosyl)-(2→3)-(6-O-benzyl-β-D-galactopyranosyl)-(1→4)-[(2,3,4-tri-O-benzyl-α-L-fucopyranosyl)-(1→3)]-2-acetamido-1-azido-6-O-benzyl-1,2-didesoxy-β-D-glucopyranose-(1_{NANA}→4_{Gal})-lacton (9) (Fig. 2):

Eine Lösung von (7) (13.5 g, 13.4 mmol), Methyl-S-(methyl-5-acetamido-2,4,7,8,9-penta-O-acetyl-3,5-dideoxy-D-glycero-D-galacto-2-nonulopyranosylat) (12.6 g, 24.2 mmol), Molekularsieb (3A) und AgOTf (7.93 g, 30.9 mmol) in Dlchlormethan/Acetonitrll (360 ml, 5:1) versetzt man bei -40°C mit Methylsulfenylbromid (MSB, 27 mmol in 31 ml 1,2-Dichlorethan). Nach 2 Stunden Rühren bei -40°C wird mit Natriumhydrogencarbonat neutralisiert und filtriert.
Es wird Dichlormethan (500 ml) zugegeben, mit gesättigter Natriumhydrogencarbonatlösung und dann mit gesättigter Natriumchloridlösung gewaschen, über MgSO₄ getrocknet, filtriert und im Vakuum eingeengt. Der Rückstand wird in Methanol (300 ml) gelöst und mit 1 M Natriummethanolatlösung in Methanol versetzt und 1 Stunde bei Raumtemperatur gerührt. Man neutralisiert mit Amberlite® IR-120 und entfernt das Lösungsmittel im Vakuum. Säulenchromatographie an Kieselgel (Dichlormethan/Methanol 25:1→10:1) liefert das 4'-Lacton (9) (7.24 g, 42.2 %) und ein Gemisch von Methylester (10) und Lacton (9) (6.0 g).
[α]_{D}²⁰ = -77.5° (1 / CH₂Cl₂); ¹H-NMR (300MHz, CDCl₃): δ = 1.05 (d, 3H, 6-H_{Fuc}), 1.78 (dd, 1H, 3-H_{Nana}), 1.95, 2.0 (2s,6H, 2 NHAc), 2.48 (dd, 1H, 3-H_{Nana}), 4.38 (ddd, 1H, 4-H_{Nana}), 5.26, 5.32 (2d, 2H, 1-H_{Fuc}, 4-H_{Gal}), 7.24-7.5 (m, 25H, Benzyl).

### Beispiel 5:

### Synthesevon N-(5-Acetamido-3,5-didesoxy-D-glycero-α-D-galacto-2-nonulopyranosylat)-(2→3)-(6-O-benzyl-β-D-galactopyranosyl)-(1→4)-[(2,3,4-tri-Obenzyl-α-L-fucopyranosyl)-(1→3)]-2-acetamido-1-amino-6-O-benzyl-1,2-didesoxy-β-D-glucopyranose-(1_{NANA}→4_{Gal})-lacton (11) (Fig. 2):

Lacton (9) (300 mg, 0.235 mmol) wird in Isopropanol (10 % Wasser, 30 ml) gelöst und mit 300 mg neutralem Raney-Nickel bei Normaldruck hydriert. Nach 1 Stunde wird filtriert und eingeengt. Das anomere Amin (11) (279 mg, 95 %) wird als amorpher Feststoff in reiner Form erhalten und unmittelbar vor seiner weiteren Umsetzung frisch hergestellt (ohne weitere Charakterisierung).

### Beispiel 6:

### Synthese des Tris-succinimidesters (VII-2):

4-(2-Carboxyethyl)-4-nitroheptandisäure (10.0 g, 36.1 mmol), N-Ethyl-N'-dimethylaminopropyl-carbodiimid-hydrochlorid (DEC) (31.1 g, 162 mmol) und N-Hydroxysuccinimid (18.7 g, 162 mmol) werden in 250 ml Dichlormethan suspendiert und 12 Stunden bei Raumtemperatur gerührt. Das kristalline farblose Produkt wird abfiltriert, zweimal mit je 100 ml Dichlormethan gewaschen und getrocknet. Ausbeute C₂₂H₂₄N₄O₁₄ (568.4): 17.7 g (86.2 %). ¹H-NMR (300 MHz, DMSO-d₆):
δ = 2.18 (m, 6H, CH₂CH₂C=O), 2.74 (m, 6H, CH₂CH₂C=O), 2.80 (s, 12H, O = CCH₂CH₂C = O).

### Beispiel 7:

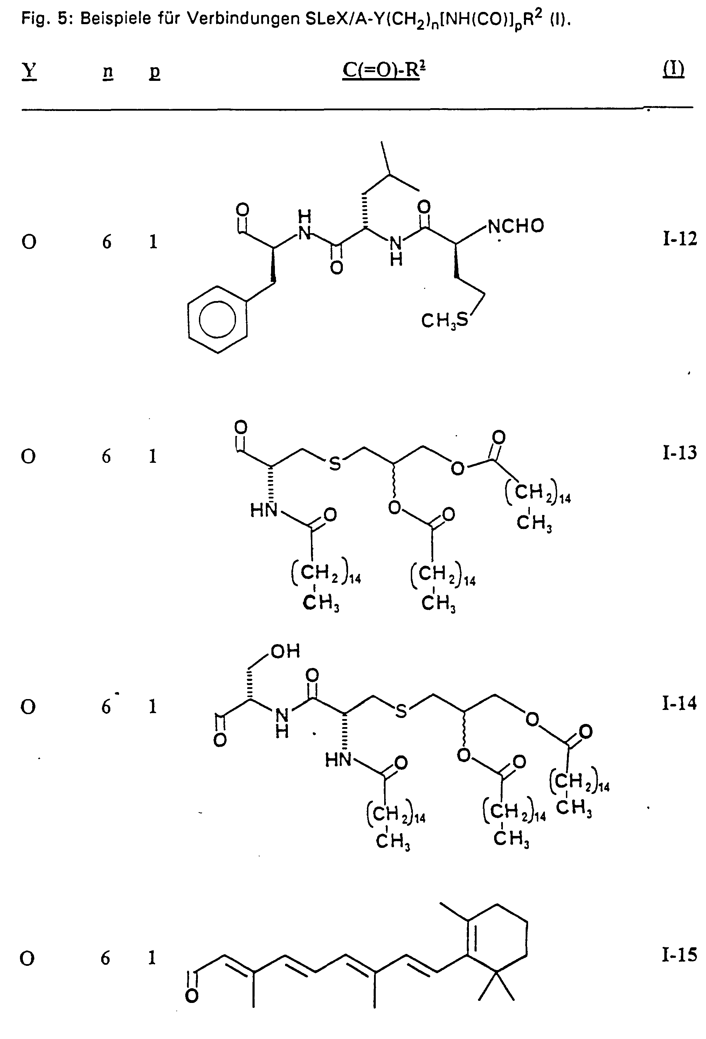

### Synthese von Tris-{2-[N-(5-Acetamido-3,5-didesoxy-D-glycero-D-galacto-2-nonulopyranosylat)-(2→3)-(β-D-galactopyranosyl)-(1→4)-[(α-L-fucopyranosyl)-(1→3)]-2-acetamido-1,2-di-desoxy-β-D-glucopyranose]-amidoethyl}-nitromethan (11-1) (Fig. 5):

Verbindungen (11) (89 mg, 0.071 mmol) und (VII, Z = OSu, m = q = 0) (10 mg, 0.019 mmol) werden in THF/Pyridin (2 ml, 1:1) über Nacht bei Raumtemperatur gerührt. Man entfernt das Lösungsmittel im Vakuum und erhält nach Säulenchromatographiean Kieselgel (Dichlormethan/Methanol 10:1) das geschützte trimere SLe^{x}-Lacton (68 mg) als Zwischenprodukt, das ohne weitere Charakterisierung weiter umgesetzt wird. Die Verbindung wird in Methanol/Dioxan (40 ml, 10:1) gelöst und nach Zugabe von Pd-Aktivkohle (84 mg) 2 h unter Normaldruck hydriert.
Nach Filtrieren, Einengen, Lactonöffnung mit 1M Natronlauge (0.5 ml) in Methanol/Wasser (40 ml/10 ml, 1 h), Neutralisieren mit Amberlite® IR-120, Entfernen der Lösungsmittel im Vakuum und Ausschlußchromatographie an Biogel® P2 erhält man Verbindung (11-1) mit der Summenformel C₁₀₃H₁₆₈N₁₀O₇₁ (2682.5) als farbloses Pulver. Ausbeute: 40 mg (78.5 %).
[α]_{D}²⁰ = -25.2 (1 / Wasser); ¹H-NMR (300 MHz, D₂O): δ = 1.05 (d, 3H, 6-H_{Fuc}), 1.66 (dd, 1H, 3-H_{Nana}), 1.86, 1.90 (2s, 6H, 2NHAc), 2.64 (dd, 1H, 3-H_{Nana}), 4.36 (d, 1H, 1-H_{Gal}), 4.95, 4.98 (2d, 2H, 1-H_{Fuc}, 1-H_{GlucNAc});
¹C-NMR (75.4 MHz, D₂O): δ = 177.9, 177.8, 177.0, 176.7 (C = O), 104.5 (1-C_{Gal}), 102.5 (2-C_{NANA}), 101.5 (1-C_{Fuc}), 95.7 (C-NO₂), 81.1, 79.9, 78.5, 78.2, 77.8, 75.8, 74.9, 74.8, 74.7, 74.5, 72.4, 72.1, 71.1, 71.0, 70.5, 70.2, 69.6, 65.5, 65.4, 64.4, 63.2, 62.4, 57.5, 54.6, 42.7, 33.2, 33.0, 25.1, 24.9, 18.2; ESI (Electrospray-Ionisierung): 1364.1 (M + 2Na)²⁺.

### Beispiel 8:

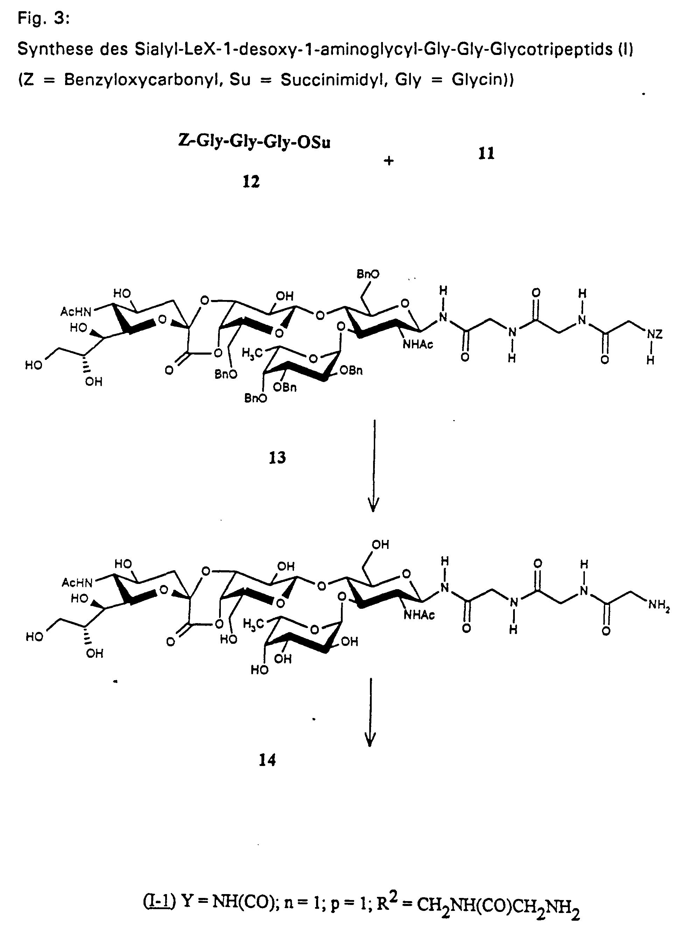

### Synthese von Benzyloxycarbonyl-Gly-Gly-Gly-N-(5-Acetamido-3,5-didesoxy-D-glycero-D-galacto-2-nonulopyranosylat)-(2→3)-(6 O-benzyl-α-L-fucopyranosyl)-(1→3)]-2-acetamido-6-O-benzyl-1,2-didesoxy-β-D-glucopyranose-(1_{NANA}→4_{Gal})-lacton (13). (Fig. 3):

Verbindung (11) (130 mg, 0.104 mmol) und Z-Gly-Gly-Gly-Su (52 mg, 0.124 mMol) werden in DMF/Dichlormethan (2ml, 1:1) gelöst. Nach 24 Stunden entfernt man das Lösungsmittel im Vakuum. Säulenchromatographie an Kieselgel (Dlchlormethan/Methanol 10:1) liefert das geschützte Zwischenprodukt (13) (98 mg, 60.5 %) mit der Summenformel C₈₀H₉₆N₆O₂₆ (1557.67) als amorphen Feststoff. ¹H-NMR (300 MHz, CD₃OD): δ = 0.99 (d, 3H, 6-H_{Fuc}), 1.69 (dd, 1H, 3-H_{Nana}), 1.80, 1.89 (2s, 6H, 2NHAc), 2.39 (dd, 1H, 3-H_{Nana}), 4.28 (ddd, 1H, 4-H_{Nana}), 4.96, 5.22 (2d, 2H, 1-H_{Fuc}, 4-H_{Gal}).

### Beispiel 9:

### Synthese von Gly-Gly-Gly-N-(5-Acetamido-3,5-dideoxy-D-glycero-D-galacto-2-nonulopyrano-sylat)-(2→3)-(β-D-galactopyranosyl)-(1→4)-[(α-L-fucopyranosyl)-(1→3)]-2-acetamido-1,2-didesoxy-β-D-glucopyranose-(1_{NANA}→4_{Gal})-lacton (14). (Fig. 3):

Verbindung (13) (90 mg, 0.059 mmol) wird in Methanol/Dioxan/Essigsäure (40 ml, 2:1:1) gelöst und nach Zugabe von Pd-Aktivkohle (50 mg) 16 Stunden unter Normaldruck mit Wasserstoff hydriert. Nach Filtration werden die Lösungsmittel im Vakuum entfernt. Ausschlußchromatographie an Biogel®-P2 liefert Verbindung (14) mit der Summenformel C₃₇H₆₁N₆O₂₄ (973.9) alsamorphen Feststoff (49 mg, 86 %), der sofort ohne weitere Charakterisierung umgesetzt wird.

### Beispiel 10:

### Synthese von Gly-Gly-Gly-N-(5-Acetamido-3,5-didesoxy-D-glycero-D-galacto-2-nonulopyrano-sylat)-(2→3)-(β-D-galactopyranosyl)-(1→4)-[(α-L-fucopyranosyl)-(1→3)]-2-acetamido-1,2-didesoxy-β-D-glucopyranose (1-1). (Fig. 3):

Man löst Verbindung (14) (45 mg, 0.046 mmol) in Methanol/Wasser (10 ml, 1:10). Mit Natronlauge wird pH 9 eingestellt und 20 Minuten gerührt. Nach Neutralisieren mit Amberlite® IR-120, Filtration, Entfernen der Lösungsmittel im Vakuum und Ausschlußchromatographie an Biogel® P2 erhält man Verbindung (1-1) (43.3 mg, 95 %) mit der Summenformel C₃₇H₆₂N₆O₂₅ (990.9) als farblosen, amorphen Feststoff.

### Beispiel 11:

### Synthese des Trimethylesters (VII-3):

4-(2-Carboxyethyl)-4-nitroheptandisäure-tris-succinimidester (VII-2) (2.0 g, 3.5 mmol), 50 ml trockenes Pyridin, 6-Aminohexansäure-methylester (2.11 g, 14.6 mmol) und 1.5 ml Triethylamin werden 5 Stunden bei 50°C gerührt. Die flüchtigen Komponenten werden im Vakuum abdestilliert und der Rückstand noch zweimal mit je 50 ml Toluol eingeengt. Das Rohprodukt wird in Essigsäureethylester aufgenommen und mit gesättigter Kochsalzlösung und mit gesättigter Natriumhydrogencarbonatlösung gewaschen. Man erhält das Produkt (VII-3) als Sirup, der ohne Reinigung weiter umgesetzt wird: Ausbeute C₃₁H₅₄N₄O₁₁ (658.8): 1.95 g (85 %). ¹H-NMR (300 MHz, DMSO-d₆):
δ = 1.10-1.60 (m, 18H, NHCH₂CH₂CH₂CH₂CH₂C = O), 2.02 (m, 12H, CH₂CH₂-C=O), 2.30 (t, 6H, CH₂COOMe), 3.00 (dt, 6H, CH₂NH), 2.57 (s, 9H, Me), 7.85 (t, 3H, NH).

### Beispiel 12:

### Synthese der Tricarbonsäure (VII-4):

Der Trimethylester (VII-3) (1.95 g, 2.9 mmol) wird mit 10 ml Methanol und 4 ml 1M NaOH bei Raumtemperatur 72 Stunden gerührt. Nach Ansäuern mit HCI auf pH = 2 wird das Produkt mit Diethylether extrahiert und nach Trocknung als Sirup erhalten: Ausbeute C₂₈H₄₈N₄O₁₁ (616.7): 1.7 g (93 %). ¹H-NMR (300 MHz, DMSO-d₆): δ = 1.10-1.60 (m, 18H, NHCH₂CH₂CH₂CH₂CH₂C=O), 2.02 (m, 12H, CH₂CH₂C=O), 2.19 (t, 6H, CH₂CO₂H), 3.00 (dt, 6H, CH₂NH), 7.85 (t, 3H, NH).

### Beispiel 13:

### Synthese des Tris-succinimidesters (VII-5):

Die Tricarbonsäure (VII-4) (235 mg, 0.38 mmol), N-Hydroxysuccinimid (218 mg, 1.9 mmol) und Dicyclohexylcarbodiimid (DCC, 281 mg, 1.4 mmol) in 10 ml THF werden über Nacht bei Raumtemperatur gerührt. Das Filtrat wird nach Abtrennung des Harnstoffs in Essigsäureethylester aufgenommen und in der Kälte erneut filtriert. Nach Waschen mit Wasser, Einengen und Trocknung erhält man das Produkt als farblosen Feststoff. Ausbeute C₄₀H₅₇N₇O₁₇ (907.9): 325 mg (94 %). ¹H-NMR (300 MHz, DMSO-d6): δ = 1.15-1.70 (m, 18H, NHCH₂CH₂CH₂CH₂), 2.02 (m, 12H, CH₂CH₂C=O), 2.65 (t, 6H, CH₂CO₂H), 2.80 (s, 12H, OSu), 3.00 (dt, 6H, CH₂NH), 7.86 (t, 3H, NH).

### Beispiel 14:

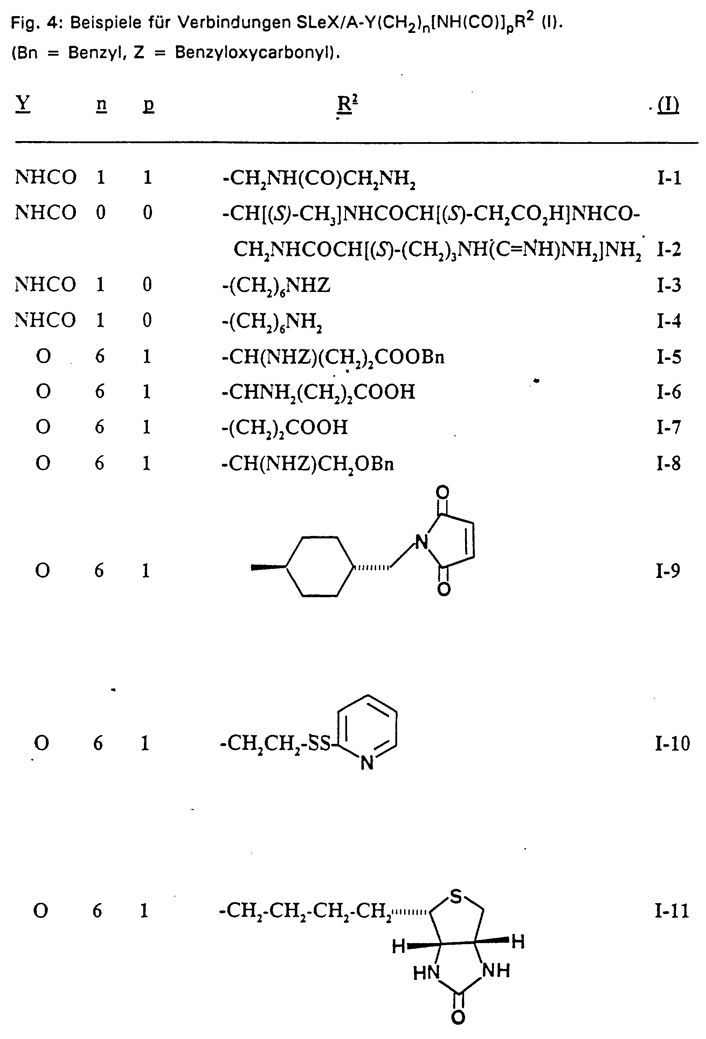

### Synthese des geschützten RGD-Ala-SLeX-Konjugats Z-Arg(Z2)-Gly-Asp(-O-benzyl)-Ala-N-(5-Acetamido-3,5-didesoxy-D-glycero-Dgalacto-2-nonulopyranosylat)-(2→3)-(β-D-galacto-pyranosyl)-(1→4)-[(α-Lfucopyranosyl)-(1→3)]-2-acetamido-1,2-didesoxy-β-D-glucopyranose-(1NANA→4Gal)-lacton als Vorstufe der Verbindung (I-2) (Fig. 4):

Verbindung (11) (300 mg, 0.24 mmol) und Z-Arg(Z2)-Gly-Asp(OBn)-Ala-OH (270 mg, 0.3 mmol) werden in DMF (4 ml) gelöst. Man gibt nacheinander 1-Hydroxybenzotriazol (41 mg, 0.3 mmol), O-(1H-Benzotriazol-1-yl)-N,N,N,N-tetramethyluroniumtetrafluoroborat (231 mg, 0.72 mmol), und N-Ethyl-diisopropylamin (78 mg, 0.6 mmol) zu und rührt anschließend 1 Stunde bei Raumtemperatur. Das Rohprodukt wird in Dichlormethan (300 ml) aufgenommen und mit Wasser, gefolgt von wässriger Natriumhydrogencarbonatlösung, gewaschen. Nach Trocknen mit MgSO₄ werden die Lösungsmittel im Vakuum entfernt. Mitteldruckchromatographie an Kieselgel (Dichlormethan/Methanol = 13/1) liefert das geschützte RGD-Ala-SLeX-Konjugat (335 mg, 65 %) als amorphen, weißen Feststoff. ¹H-NMR (300 MHz, CD₃OD): δ = 1.08 (d, 3H, 6-H_{Fuc}), 1.3 (d, 3H, b-H_{Ala}), 1.78 (dd, 1H, 3-H_{Nana}), 1.91, 2.0 (2s, 6H, 2 NHAc), 2.49 (dd, 1H, 3-H_{Nana}), 2.79, 3.01 (2dd, 2H, b-HAsp, b-H_{Asp}), 4.46 (d, 1H, 1-H_{Gal}), 5.3 (d, 1H, 1-H_{Fuc}).

### Beispiel 15:

### Synthese von Arg-Gly-Asp-N-(5-Acetamido-3,5-didesoxy-D-glycero-D-galacto-2-nonulo-pyranosylat)-(2→3)-(b-D-galactopyranosyl)-(1→4)-[(α-L-fucopyranosyl)-(1→3)]-2-acetamido-1,2-didesoxy-β-D-glucopyranose (I-2) (Fig. 4):

Das geschützte Vorprodukt aus Beispiel 14 (125 mg, 0.059 mmol) wird in Methanol/Dioxan/Essigsäure (40 ml, 2:1:1) gelöst und nach Zugabe von Pd-Aktivkohle (125 mg) 16 Stunden unter Normaldruck mit Wasserstoff hydriert. Nach Filtration werden die Lösungsmittel im Vakuum entfernt. Die Lactonöffnung erfolgt mit 1 M Natronlauge (0.5 ml) in Methanol/Wasser (20 ml, 1:10) bei pH 8.5. Nach Neutralisieren mit Amberlite® IR-120, Filtration, Entfernen der Lösungsmittel im Vakuum und Ausschlußchromatographie an Biogel® P2 erhält man Verbindung (I-2) (84 mg, 58 %) der Summenformel C₄₆H₇₈N₁₀O₂₈ (1219.31) als farblosen, amorphen Feststoff ¹H-NMR (300 MHz, D₂O): δ = 1.18 (d, 3H, 6-H_{Fuc}), 1.37 (d, 3H, b-H_{Ala}), 1.8 (dd, 1H, 3-H_{Nana}), 1.99, 2.04 (2s, 6H, 2 NHAc), 2.6-2.9 (3dd, 3H, b-H_{Asp}, b-H_{Asp}, 3-H_{Nana}), 4.52 (d, 1H, 1-H_{Gal}), 5.1 (d, H, 1-H_{Fuc}); FAB-MS (Fast Atom Bombardement): 1219.5 (MH)⁺.

### Beispiel 16:

### Synthese von (1-5) mit SLeX-Konfiguration (Fig. 4):

Die Verbindung wird aus dem SLeX-Tetrasaccharid (III; n = 6) der Formel C₃₇H₆₅N₃O₂₃ (919.9), das nach der Vorschrift in EP 0 601 417 erhältlich ist (18.5 mg, 0.020 mmol), und dem käuflichen (Firma Bachem) Aktivester Z-Glu(OBn)ONp (9.9 mg, 0.020 mmol) durch Rühren in Pyridin (15 ml) für 24 Stunden bei Raumtemperatur hergestellt. Nach DC-Kontrolle (BuOH/Aceton/HOAc/Wasser 35/35/7/23) entsteht das im Vergleich zum Kohlenhydrat-Edukt lipophilere Produkt mit einem RF-Wert von 0.76 (Edukt: RF 0.21). Nach Konzentrieren auf 1 ml wird das Produkt mit 30 ml Essigsäureethylester ausgerührt und durch Zentrifugieren isoliert. Das Ausrühren und Zentrifugieren wird noch zweimal wiederholt. Das Rohmaterial (17 mg) wird über eine Biogel®-Säule (18 x 170 mm) mittels Wasser eluiert und gefriergetrocknet.
Ausbeute: 16.0 mg (63 %) der Verbindung C₅₇H₈₄N₄O₂₈ (1273.3). Weitere Umsetzung siehe Beispiel 17.

### Beispiel 17:

### Synthese von (I-6) mit SLeX-Konfiguration (Fig. 4):

Die Verbindung wird aus dem oben beschriebenen, geschützten Zwischenprodukt (16.8 mg, 0.013 mmol) durch Hydrierung mit Pd/Aktivkohle in 8 ml Methanol hergestellt. Ausbeute der Verbindung C₄₂H₇₂N₄O₂₆ (1049.03). FAB-Massenspektrum (3-Nitrobenzylalkohol/NaCl):
m/e = 1049.3 [MH]⁺, 1073.3 [M+Na]⁺.

### Beispiel 18:

### Synthese von (1-7) mit SLeX-Konfiguration (Fig. 4):

SLeX-Tetrasaccharid (III; n = 6) der Formel C₃₇H₆₅N₃O₂₃ (919.9), hergestellt nachder Vorschrift in EP 0 601 417 (23.9 mg, 0.026 mmol), und Bernsteinsäure-anhydrid (2.6 mg, 0.026 mmol) in 10 ml Pyridin werden 12 Stunden bei 10°C umgesetzt (DC-Kontrolle). Nach Einengen wird das Produkt aus Wasser gefriergetrocknet. Ausbeute der Verbindung C₄₁H₆₉N₃O₂₆ (1019.03): 25.2 mg (92 %).
FAB-Massenspektrum (3-Nitrobenzylalkohol): m/e = 1018.4 [M-H]⁻.

### Beispiel 19:

### Synthese des SLeX-Ser-Konjugates (I-8) (Fig. 4):

SLeX-Tetrasaccharid (III; n = 6 in der Lactonform1_{NANA},→4_{Gal}) der Summenformel C₃₇H₆₃N₃O₂₂ (901.9), hergestellt nach der Vorschrift in EP 0 601 417 (70.0 mg, 0.077 mmol), und Z-Ser(OBn)-OSu (36.0 mg, 0.084 mmol) in 20 ml Pyridin werden 20 Stunden bei Raumtemperatur gerührt (DC-Kontrolle). Nach Einengen wird das Lacton-Zwischenprodukt in 10 ml Methanol mit 1 N NaOH bei pH = 12 verseift (DC-Kontrolle). Nach Neutralisation mit saurem lonenaustauscher wird eingeengt und über Sephadex® LH-20 (35 x 70 mm) mit Methanol chromatographiert. Die Verbindung C₅₅H₈₂N₄O₂₇ (1231.3) wird in einer Ausbeute von 78 mg (90.5 %) erhalten und kann ganz analog wie oben in Beispiel 16 für das SLeX-Glu-Konjugat beschrieben durch katalytische Hydrierung entschützt werden. Aus 53 mg (0.043 mmol) erhält man so das freie SLeX-Ser-Konjugat C₄₀H₇₀N₄O₂₅ (1007.0) in einer Ausbeute von 62 mg (80 %) bezogen auf das SLeX-Tetrasaccharid (III; n = 6 in der Lactonform1_{NANA}→4_{Gal}).

### Beispiel 20:

### Synthese des Pam₃Cys-Ser-SLeX-Konjugates(I-14) (siehe Fig. 5):

Das nach Beispiel 19 hergestellte SLeX-Ser-Konjugat (I-8) der Summenformel C₄₀H₇₀N₄O₂₅ (20 mg, 0.02 mmol) wird mit Pam₃Cys-OSu (30.2 mg, 0.03 mmol; hergestellt nach Int. J. Peptide Protein Res. 37, 1991, 46) in 5 ml Pyridin 24 Stunden bei Raumtemperatur umgesetzt. Nach Einengen wird der Rückstand an Toyopearl® HW-40 mit dem Laufmittel Methanol/Dichlormethan (1/1) chromatographiert. Man erhält das Pam₃Cys-Ser-SLeX-Konjugat (1-14) der Summenformel C₉₄H₁₇₁N₅O₃₁S (1899.4). Ausbeute: 25 mg (66 %). FAB-MS (Glycerin, KCl): m/e = 1897.1.

Das mit einer Benzylschutzgruppe am Serin versehene Derivat dieser Verbindung wird ganz analog erhalten, indem die Hydrierung der Z- und Benzyl-geschützten Vorstufe aus Beispiel 19 nach selektiver Abspaltung der Z-Schutzgruppe (DC-Kontrolle) abgebrochen und das Zwischenprodukt analog wie oben beschrieben mit Pam₃Cys-OSu umgesetzt wird. Man erhält das Benzylderivat von (I-14) mit der Summenformel C₁₀₁H₁₇₇N₅O₃₁S (1989.6).
FAB-MS (Glycerin, KCI): m/e = 1988.1. ¹H-NMR (500 MHz, CD₃OD/CDCl₃ = 2/1): δ = 0.90 (3t, 9 H, CH₃-Pam), 1.17 (d, 3H, 6-H_{Fuc}), 1.20-1.70 (m, ca. 86H, CH₂), 1.74 (m, 1H, 3-H_{Nana/ax}), 1.96, 2.03 (2s, 6H, 2 NAc), 2.20-2.40 (m, 6H, C₁₄H₂₉CH₂COO), 2.68-2.91 (m, 3H, 1xCH₃S und 3-H_{Nana/äqu}), 3.33 (m, 2 H, CH₂CH₂NHCO), 5.04 (d, 1H, 1-H_{Fuc}), 5.18 (m, 1H, C₁₄H₂₉CH₂CO-OCH), 7.30 (m, 5H, Phe). ¹³C-NMR (125.7 MHz, CD₃OD/CDCl₃ = 2/1): δ = 103.42, 101.82, 100.23, 99.41 (1-C_{Gal}, 1-C_{GlcNAc}, 2-C_{NANA}, 1-C_{Fuc}), 16.31(6-C_{Fuc}), 14.37 (16-C-Pam, 3x).

### Beispiel 21:

### Synthese des Konjugates (I-9) mit SLeX-Konfiguration (Fig. 4):

SLeX-Tetrasaccharid (III; n = 6) der Formel C₃₇H₆₅N₃O₂₃ (919.9), hergestellt nachder Vorschrift in der EP 0 601 417 (20.0 mg, 0.022 mmol), wird in 10 ml Pyridin gelöst und mit 7.3 mg (0.022 mmol) Succinimidyl-4-(N-maleimidomethyl)cyclohexan-1-carboxylat (SMCC, Firma Pierce) versetzt. Nach 20 Stunden Rühren bei Raumtemperatur (DC-Kontrolle) wird das Produkt mit Essigsäureethylester ausgefällt und durch Zentrifugieren isoliert. Nach Waschen mit Essigsäureethylester wird das Produkt in Wasser gelöst, steril filtriert und gefriergetrocknet. Man erhält 20.3 mg (81 %) der Verbindung (I-9) mit der Summenformel C₄₉H₇₈N₄O₂₆ (1139.17). FAB-MS (3-Nitrobenzylalkohol): m/e = 1137.5 [M-H]⁻.

Setzt man das SLeX-Tetrasaccharid (III; n = 6 in der Lactonform1_{NANA}→4_{Gal}) der Summenformel C₃₇H₆₃N₃O₂₂ (901.9), hergestellt nach der Vorschrift in EP 0 601 417, analog mit SMCC in Pyridin um und spaltet das Lacton analog wie in Beispiel 19 beschrieben mit NaOH in Methanol/Wasser, so wird der Maleinimid-Ring geöffnet und man erhält nach Ansäuern und Reinigung des Produktes mittels Chromatographie an Biogel® das Hydrolyseprodukt der Summenformel C₄₉H₈₀N₄O₂₇ (1157.17). FAB-MS (Glycerin, KCI): m/e = 1155.6 [M-H]⁻. MS mit Electrospray-lonization (ESI, Glycerin-Matrix): m/e = 1158 [M-H]⁺; m/e = 1180 [M+Na]⁺.
Die Verbindung hydrolysiert in wässriger Lösung langsam weiter unter Abspaltung von Maleinsäure und es entsteht C₄₅H₇₈N₄O₂₄ (1059.12). FAB-MS (Glycerin): m/e = 1057.6 [M-H]⁻.

### Beispiel 22:

### Synthese des Konjugates (I-10) mit SLeX-Konfiguration (Fig. 4):

SLeX-Tetrasaccharid (III; n = 6) der Formel C₃₇H₆₅N₃O₂₃ (919.9), hergestellt nach EP 0 601 417 (21.3 mg, 0.023 mmol), wird in 10 ml Pyridin gelöst und mit 7.24 mg (0.023 mmol) N-Succinimidyl-3-(2pyridyldithio)propionat (SPDP, Firma Pierce) versetzt. Nach 16 Stunden Rühren bei Raumtemperatur (DC-Kontrolle) wird das Produkt mit Essigsäureethylester ausgefällt und durch Zentrifugieren isoliert. Nach Waschen mit Essigsäureethylester wird das Produkt in Wasser gelöst, sterilfiltriert und gefriergetrocknet. Man erhält 20.2 mg (78 %) der Verbindung (I-10) mit der Summenformel C₄₅H₇₂N₄O₂₄S₂ (1117.2). FAB-MS (3-Nitrobenzylalkohol): m/e = 1115.5[M-H]⁻.

### Beispiel 23:

### Synthese des Konjugates (I-11) mit SLeX-Konfiguration (Fig. 4):

SLeX-Tetrasaccharid (III; n = 6) der Formel C₃₇H₆₅N₃O₂₃ (919.9), hergestellt nachder Vorschrift in EP 0 601 417 (22.0 mg, 0.024 mmol), wird in 10 ml Pyridin gelöst und mit 8.2 mg (0.024 mmol) NHS-Biotin (Firma Pierce) versetzt. Nach 48 Stunden Rühren bei Raumtemperatur (DC-Kontrolle) wird das Lösungsmittel im Vakuum entfernt und das Produkt über Biogel® gereingt. Man erhält 23 mg (83 %) der Verbindung (I-11) mit der Summenformel C₄₇H₇₉N₅O₂₅S (1146.22). FAB-MS (3-Nitrobenzylalkohol): m/e = 1144.4 [M-H]⁻.

Das Streptavidin-Konjugat dieser Verbindung wird wie folgt erhalten: Streptavidin (10 mg von der Firma Pierce No. 21125) wird in 5 ml Wasser gelöst und mit 1.54 mg (1.34 µmol) (I-11) versetzt. Nach 1 Stunde wird das Produkt über Biogel® P2 gereinigt. Nach Gefriertrocknung erhält man 13.7 mg farbloses SLeX-Biotin-Streptavidin-Konjugat.

### Beispiel 24:

### Synthese des Konjugates (I-12) mit SLeX-Konfiguration (Fig. 5):

N-Formyl-Met-Leu-Phe-OH (100 mg, 0.23 mmol), N-Hydroxysuccinimid (29 mg, 0.25 mmol), Dicyclohexylcarbodiimid (52 mg, 0.25 mmol) werden 12 Stunden in 3 ml DMF gerührt. Nach Einengen im Vakuum wird der Rückstand in 5 ml Pyridin aufgenommen und mit SLeX-Tetrasaccharid (III; n = 6 in der Lactonform1_{NANA}→4_{Gal}) der Summenformel C₃₇H₆₃N₃O₂₂ (901.9), hergestellt nach der Vorschrift in EP 0 601 417 (90 mg, 0.099 mmol), und mit Diisopropylethylamin (0.5 ml) versetzt.
Nach 14 Rühren bei Raumtemperatur wird das Pyridin im Vakuum entfernt. Der Rückstand wird in 5 ml Methanol und 1 ml Wasser gelöst und mit 1 N NaOH auf pH = 11.5 gestellt. Nach 1 Stunde wird mit 0.1 N HCI neutralisiert und das Produkt an Toyopearl® HW-40/Methanol gereinigt. Man erhält 56 mg (42 %) des Produktes (1-12) mit der Summenformel C₅₈H₉₄N₆O₂₇S (1339.46).

MS mit Electrospray-lonization (ESI, Glycerin-Matrix): m/e = 1361.6 [M+Na]⁺. ¹H-NMR (500 MHz, CD₃OD): δ = 0.85, 0.91 (2d, 6H, CH₃-Leu), 1.16 (d, 3H, 6-H_{Fuc}), 1.73 (m, 1H, 3-H_{Nana}), 1.95, 2.00 (2s, 6H, 2 NAc), 2.08 (s, 3H, CH₃S), 2.50 (m, CH₂S), 2.87 (m, 1H, 3-H_{Nana}), 3.29 (m, 2 H, CH₂CH₂NHCO), 4.04 (m, 1H, 3-H_{Gal}), 5.05 (d, 1H, 1-H_{Fuc}), 7.22 (m, 5H, Phe), 8.10 (s, 1H, NHCHO).

### Beispiel 25:

### Synthese des Pam₃Cys-SLeX-Konjugates(I-13) (Fig. 5):

SLeX-Tetrasaccharid (III; n = 6) der Formel C₃₇H₆₅N₃O₂₃ (919.9), hergestellt nachder Vorschrift in EP 0 601 417 (38 mg, 0.041 mmol), wird mit Pam₃Cys-OSu (60.4 mg, 0.06 mmol; Int. J. Peptide Protein Res. 37, 1991, 46) in 10 ml Pyridin 24 Stunden bei Raumtemperatur umgesetzt. Nach Einengen wird das Produkt an Toyopearl® HW-40 mit Dichlormethan/Methanol (1/1) gereinigt. Man erhält das Pam₃Cys-SLeX-Konjugat (I-13) der Summenformel C₉₁H₁₆₆N₄O₂₉S (1812.39).
Ausbeute: 40 mg (54 %). FAB-MS (Glycerin): m/e = 1812.4 [MH]⁺.

### Beispiel 26:

### Synthese von Pam₃-Cys-Ala-Gly-SLeX (II-17)

Aus 50 mg (0.048 mmol) Pam₃Cys-Ala-Gly-OH (Int. J. Peptide Protein Res. 37, 1991, 46) wird analog wie in Beispiel 24 beschrieben der OSu-Ester hergestellt und in 5 ml Pyridin mit 30 mg (0.033 mol) SLeX-Tetrasaccharid (III; n = 6) der Formel C₃₇H₆₅N₃O₂₃ (919.9), hergestellt nach der Vorschrift in EP 0 601 417, 30 Minuten bei 65°C umgesetzt. Nach Einengen wird das Produkt an Toyopearl® HW-40 mit dem Laufmittel Methanol/Dlchlormethan (1/1) chromatographiert. Man erhält die Verbindung (I-17) der Summenformel C₉₆H₁₇₄N₆O₃₁S (1940.5). Ausbeute: 37 mg (58 %). FAB-MS (Glycerin): m/e = 1938.1 [M-H]⁻.

### Beispiel 27:

### Synthese des Vitamin A-Konjugates mit SLeX-Konfiguration (1-15) (Fig. 5):

Vitamin A-Säure (79.5 mg, 0.265 mmol) wird in 5 ml Dichlormethan und 1 ml THF mit HOSu (33.5 mg, 0.292 mmol) und DCC (57 mg, 0.278 mmol) 12 Stunden umgesetzt (DC-Kontrolle). Das Filtratwird eingeengt und getrocknet. Ausbeute Rohprodukt OSu-Ester: 117 mg. Dieses Rohpodukt (24 mg, 0.06 mmol) wird in 5 ml Pyridin mit 54 mg (0.057 mmol) SLeX-Tetrasaccharid (III; n = 6 in der Lactonform 1_{NANA}→4_{Gal}) der Summenformel C₃₇H₆₃N₃O₂₂ (901.9), hergestellt nach der Vorschrift in EP 0 601 417, und Diisopropylethylamin (0.5 ml) unter Argon 16 Stunden bei Raumtemperatur und dann 8 Stunden bei 40°C umgesetzt. Nach Einengen der Lösung wird das Produkt aus Dichlormethan ausgerührt und abfiltriert. Der Feststoff wird in 3 ml Wasser gelöst und mit 0.1 N NaOH auf pH = 11.5 gestellt. Nach 2 Stunden Rühren wird neutralisiert und das Produkt mit präparativer HPLC an Eurosil® Bioselect®-100 RP-18 (250 x 20 mm) mit 20 bis 60 % Acetonitril/Wasser gereinigt. Man erhält 29 mg (42 %) (I-15) mit der Summenformel C₅₇H₉₁N₃O₂₄ (1202.4). MS Electrospray-lonization (ESI, Glycerin-Matrix): m/e = 1203 [MH]⁺; als Nebenkomponenten sind oxidierte Spezies zu erkennen: m/e = 1219, 1235. ¹H-NMR (300 MHz, CD₃OD): δ = 1.02 (s, 6 H, 2CH₃), 1.15 (d, 3H, 6-H_{Fuc}), 1.72 (s, 3H, CH₃), 1.95, 2.00 (2s, 6H, 2 NAc), 1.98 (s, 3H, CH₃), 2.29 (s, 3H, CH₃), 2.87 (dd, 1H, 3-H_{Nana/equ}), 3.20 (m, 2 H, CH₂CH₂NHCO), 4.41, 4.51 (2d, je 1-H_{Gal}, 1-H_{GlcNAc}), 5.05 (d, 1H, 1-H_{Fuc}), 6.05-6.40 (m, ca. 5H, CH =), 6.05 (dd, J = 11 Hz, J = 15 Hz, 1H, CH=).

### Beispiel 28:

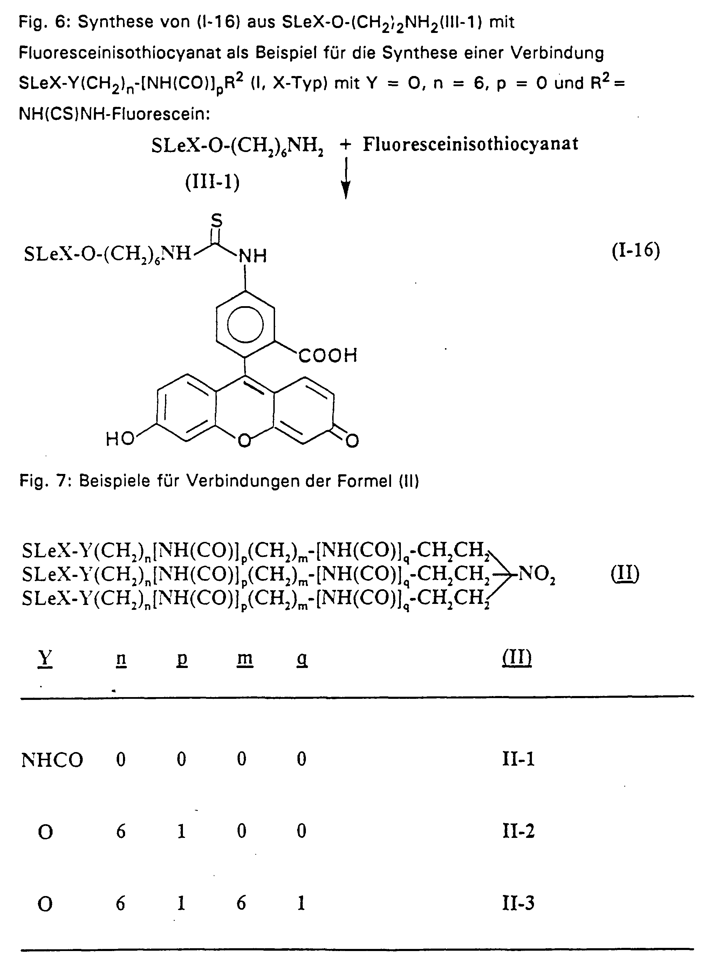

### Synthese des Fluorescein-Konjugates (I-16) (Fig. 6):

1.30 g (1.44 mmol) SLeX-Tetrasaccharid (III; n = 6 in der Lactonform1_{NANA}→4_{Gal}:C₃₇H₆₃N₃O₂₂ = 901.9), hergestellt nach der Vorschrift in EP 0 601 417, und 0.57 g (1.46 mmol) Fluoresceinisothiocyanat (Fluka No. 46951) in 180 ml Pyridin werden 20 Stunden bei Raumtemperatur gerührt. Nach Einengen zur Trockne wird der Rückstand in 3 ml Wasser gelöst, mit 0.1 N NaOH auf pH = 12 gestellt und nach 1 Stunde Rühren bei Raumtemperatur mit HCl/Wasser neutralisiert. Das Produkt wird an Biogel® P2 gereinigt. Ausbeute: 1.67 g (88.6 %) Feststoff (orange).
Summenformel C₅₈H₇₆N₄O₂₈S (1309.3). FAB-MS (Glycerin/MeOH): m/e = 1309.0 [MH]⁺, 1330.8 [M+Na]⁺.

### Beispiel 29:

### Synthese der trivalenten Verbindung (II-2) (Fig. 7):

30 mg (0.033 mmol) SLeX-Tetrasaccharid (III; n = 6 in der Lactonform1_{NANA}→4_{Gal}: C₃₇H₆₃N₃O₂₂ = 901.9), hergestellt nach der Vorschrift in EP 0 601 417, und 5.4 mg (0.01 mmol) Tris-succinimid (VII-2), hergestellt nach Beispiel 6, werden in 7 ml Pyridin 7 Stunden bei 60°C gerührt. Nach Einengen wird der Rückstand in 3 ml Wasser gelöst, mit 1 N NaOH auf pH = 12 gestellt, nach 1 Stunde mit saurem lonenaustauscher neutralisiert und das Produkt über Biogel® P4/Wasser gereinigt. Man erhält 24 mg (84 %) der Verbindung (II-2) mit der Summenformel C₁₂₁H₂₀₄N₁₀O₇₄ (2983.0) als farbloses Pulver. MS Electrospray-lonization (ESI): m/e = 993.3 [M-3H]³⁻, 1490.4 [M-2H]²⁻.

¹H-NMR (500 MHz, D₂O): d =1.17 (d, 3H, J = 6.5 Hz, 6-H_{Fuc}), 1.28-1.38 (m, 4H, NCH₂CH₂-CH₂CH₂CH₂CH₂O), 1.45-1.59 (m, 4H, NCH₂CH₂CH₂CH₂CH₂CH2O), 1.80 (pseudo-t, J = 12 Hz, 1H, 3-H_{Nana/ax}), 2.02, 2.04 (2s, 6H, 2 NAc), 2.20-2.30 (m, 4H, CH₂CH₂CNO₂), 2.77 (dd, 1H, J = 4.5 Hz, J = 12.5 Hz, 3-H_{Nana/equ}), 3.16 (t, 2 H, J = 6.5 Hz, CH₂CH₂NHCO), 3.53 (dd, 1H, J = 7.5 Hz, J = 9.5 Hz, 2-H_{Gal}), 4.09 (dd, 1H, J = 3.0 Hz, J = 9.5 Hz, 3-H_{Gal}), 4.53 (2d, 2H, 1-H_{Gal}, 1-H_{GlcNAc}), 4.83 (m, 1H, H-5_{Fuc}), 5.11 (d, 1H, J = 4.0 Hz, 1-H_{Fuc}).
¹³C-NMR (125.7 MHz, D₂O): δ = 175.06, 174.14, 174.12, 173.93 (C = O), 101.66 (1-C_{Gal}), 101.01 (1-C_{GlcNAc}), 99.70 (2-C_{NANA}), 98.65 (1-C_{Fuc}), 75.68, 75.29, 74.92, 74.89(3-C_{Gal}, 5-C_{GlcNAc}, 3-C_{GlcNAc}, 5-C_{Gal}), 73.39, 72.94 (4-C_{GlcNAc}, 6-C_{Nana}), 71.94, 71.88 (4-C_{Fuc},8-C_{Nana}), 70.52 (Spacer-CH₂O), 69.30, 69.22 (2-C_{Gal}, 3-C_{Fuc}), 68.36, 68.14 (4-C_{Nana}, 7-C_{Nana}), 67.75, 67.34, 66.71 (2-C_{Fuc}, 4-C_{Gal}, 5-C_{Fuc}), 62.62 (9-C_{Nana}), 61.51 (6-C_{Gal}), 59.70 (6-C_{GlcNAc}), 55.17 (2-C_{GlcNAc}), 51.73 (5-C_{Nana}), 39.81, 39.46 (Spacer-CH₂NH, 3-C_{Nana}), 30.64, 30.18 [C(NO₂)CH₂CH₂], 28.55, 28.25, 25.75, 24.78 (NCH₂CH₂CH₂CH₂CH₂CH₂O), 22.31, 22.07 (NAc), 15.31 (6-C_{Fuc}).

### Beispiel 30:

### Synthese der trivalenten Verbindung (II-3) (Fig. 7):

98 mg (0.108 mmol) SLeX-Tetrasaccharid (III; n = 6 in der Lactonform1_{NANA}→4_{Gal}:C₃₇H₆₃N₃O₂₂ = 901.9), hergestellt nach der Vorschrift in EP 0 601 417, und 30 mg (0.033 mmol) Tris-succinimid (VII-5), hergestellt nach Beispiel 13, werden in 10 ml Pyridin 10 Stunden bei 60°C gerührt. Nach Einengen wird der Rückstand in 10 ml Wassergelöst (trüb löslich), mit 1 N NaOH auf pH = 12 gestellt, nach 1 Stunde mit saurem lonenaustauscher neutralisiert und das Produkt über Biogel® P4/Wasser gereinigt. Man erhält 17 mg (15 % bezogenauf VII-5 und 14 % bezogen auf das Tetrasaccharid) der Verbindung (II-2) mit der Summenformel C₁₃₉H₂₃₇N₁₃O₇₇ (3322.4) als farbloses Pulver. MS Electrospray-Ionization (ESI): m/e = 1106.5 [M-3H]³⁻; von der bivalenten Verbindung mit der mittleren Molmasse 2420 findet man im MS nur Spuren: m/e = 805.8 [M-3H]³⁻

¹H-NMR (500 MHz, D₂O): δ = 1.13 (d, 3H, J = 6.5 Hz, 6-H_{Fuc}), 1.22-1.33 (m, 6H, NCH₂CH₂-CH₂CH₂CH₂CH₂₀ und NHCOCH₂CH₂CH₂CH₂CH₂CO), 1.40-1.60 (m, 8H, NCH₂CH₂CH₂-CH₂CH₂CH₂O und NHCOCH₂CH₂CH₂CH₂CH₂CO), 1.75 (pseudo-t, J = 12 Hz, 1H, 3-H_{Nana/ax}), 1.97, 1.99 (2s, 6H, 2 NAc), 2.15-2.27 (m, 6H, CH₂CH₂CNO₂ und CH₂CH₂CH₂CONH), 2.74 (dd, 1H, J = 4.5 Hz, J = 12.5 Hz, 3-H_{Nana/equ}), 3.13 (2t, 4H, J = 6.5 Hz, 2xCH₂NHCO), 4.04 (dd, 1H,J = 3.0 Hz, J = 9.5 Hz, 3-H_{Gal}), 4.48 (2d, 2H, 1-H_{Gal}, 1-H_{GlcNAc}), 4.78 (m, 1H,H-5_{Fuc}), 5.07 (d, 1H, J = 4.0 Hz, 1-H_{Fuc}).
¹³C-NMR (125.7 MHz, D₂O): δ = 176.50, 174.92, 173.97, 173.90, 173.79 (CO), 101.52 (1-C_{Gal}), 100.88 (1-C_{GlcNAc}), 99.56 (2-C_{NANA}), 98.51 (1-C_{Fuc}), 93.39 (CNO₂), 75.55, 75.16, 74.79, 74.76 (3-C_{Gal}, 5-C_{GlcNAc}, 3-C_{GlcNAc}, 5-C_{Gal}), 73.25, 72.81 (4-C_{GlcNAc}, 6-C_{Nana}), 71.95, 71.80 (4-C_{Fuc},8-C_{Nana}), 70.36 (Spacer-CH₂O), 69.42, 69.17, 68.21, 68.01, 67.62, 67.21, 66.58, (2-C_{Gal}, 3-C_{Fuc}, 4-C_{Nana}, 7-C_{Nana}, 2-C_{Fuc}, 4-C_{Gal}, 5-C_{Fuc}), 62.49 (9-C_{Nana}), 61.38, 59.56, (6-C_{Gal}, 6-C_{GlcNAc}), 55.75 (2-C_{GlcNAc}), 51.60 (5-C_{Nana}), 39.67, 39.17, 39.12 (2x Spacer-CH₂NH, 3-C_{Nana}), 35.58 (OC₆H₁₂NH-COCH₂), 30.55, und NHCOCH₂CH₂CH₂CH₂CH₂CO), 22.17, 21.93 (Nac), 30.06 [C(NO₂)CH₂CH₂], 28.45, 28.26, 27.79, 25.64, 25.40, 24.99, 24.68, (NCH₂CH₂CH₂CH₂CH₂CH₂O 15.18 (6-C_{Fuc}).

### Beispiel 31:

### Leukozyten-Adhäsion - Prüfung der Wirksamkeit in vivo

Bei entzündlichen Prozessen und anderen, die Zytokine aktivierenden Zuständen spielt die Gewebszerstörung durch einwandernde oder die Mikrozirkulation blockierende Leukozyten eine entscheidende Rolle. Die erste und für den weiteren Krankheitsprozeß entscheidende Phase ist die Aktivierung von Leukozyten innerhalb der Blutbahn, insbesondere im prä- und postkapillären Bereich. Dabei kommt es, nachdem die Leukozyten den Axialstrom des Blutes verlassen haben, zu einem ersten Anheften der Leukozyten an der Gefäßinnenwand, d.h. am Gefäßendothel. Alle darauf folgenden Leukozyteneffekte, d.h. die aktive Durchwanderung durch die Gefäßwand und die anschließende orientierte Wanderung im Gewebe, sind Folgereaktionen (Harlan, J.M., Leukocyte-endothelial interaction, Blood 65, 513-525, 1985).

Diese rezeptorvermittelte Interaktion von Leukozyten und Endothelzellen wird als ein initiales Zeichen des Entzündungsprozesses angesehen. Neben den schon physiologisch exprimierten Adhäsionsmolekülen kommt es unter der Einwirkung von Entzündungsmediatoren (Leukotriene, PAF) und Zytokinen (TNF-alpha, Interleukinen) zur zeitlich gestuften, massiven Expression von Adhäsionsmolekülen auf den Zellen. Sie werden derzeit in drei Gruppen eingeteilt: 1. Immunglobulin-Gensuperfamilie, 2. Integrine und 3. Selektine. Während die Adhäsion zwischen Molekülen der Ig-Gensuperfamilie und den Protein-Protein-Bindungen abläuft, stehen bei der Kooperation zwischen Selektinen Lektin-Kohlehydrat-Bindungen im Vordergrund (Springer, T.A., Adhesion receptors of the immune system. Nature 346, 425-434, 1990; Huges, G., Cell adhesion molecules - the key to an universal panacea, Scrips Magazine 6, 30-33, 1993; Springer, T.A., Traffic signals for lymphocyte recirculation and leukocyte emigration; The multistep paradigm. Cell 76, 301-314, 1994).

### Methode:

Die induzierte Adhäsion von Leukozyten wird mit einer intravitalmikroskopischen Untersuchungstechnik im Mesenterium der Ratte quantifiziert (Atherton A. and Born G.V.R., Quantitative investigations of the adhesiveness of circulating polymorphnuclear leukocytes to blood vessel walls. J. Physiol. 222, 447-474, 1972; Seiffge, D. Methoden zur Untersuchung der Rezeptor-vermittelten Interaktion zwischen Leukozyten und Endothelzellen im Entzündungsgeschehen, in: Ersatz- und Ergänzungsmethoden zu Tierversuchen in der biomedizinischen Forschung, Schöffl, H. et al., (Hrsg.) Springer, 1995 (im Druck)). Unter Inhalations-Äthernarkose wird eine Dauernarkose durch intramuskuläre Injektion von Urethan (1,25 mg/kg KG) eingeleitet. Nach Freipräparation von Gefäßen (V. femoralis zur Injektion von Substanzen und A. carotis zur Blutdruckmessung) werden Katheter in diese eingebunden. Danach wird das entsprechende transparente Gewebe (Mesenterium) nach den in der Literatur bekannten Standardmethoden freigelegt und auf dem Mikroskoptisch ausgelagert und mit 37°C warmen Paraffinöl überschichtet (Menger, M.D. and Lehr, H., A. Scope and perspetives of intravital microscopy-bridge over from in vitro to in vivo, Immunology Today 14, 519-522, 1993). Die Applikation der Testsubstanz erfolgt i.v. am Tier (10mg/kg). Die experimentelle Erhöhung der Blutzell-Adhäsion wird durch systemische Verabreichung von Lipopolysaccharid (LPS, 15 mg/kg) 15 Minuten nach Applikation aus Testsubstanz durch Zytokin-Aktivierung ausgelöst (Foster S.J., Mc Cormick L.M., Ntolosi B.A. and Campbell D., Production of TNF-alpha by LPS-stimulated murine, rat and human blood and its pharmacological modulation, Agents and Actions 38, C77-C79, 1993, 18.01.1995). Die dadurch verursachte erhöhte Adhäsion von Leukozyten am Endothel wird direkt vitalmikroskopisch oder mit Hilfe von Fluoreszenzfarbstoffen quantifiziert. Alle Meßvorgänge werden per Videokamera aufgenommen und auf einem Videorekorder gespeichert. Über einen Zeitraum von 60 Minuten wird alle 10 Minuten die Anzahl der rollenden Leukozyten (d.h. alle sichtbar rollenden Leukozyten, die langsamer als die strömenden Erythrozyten sind) und die Anzahl an haftenden Leukozyten am Endothel (Verweildauer länger als 5 Sekunden) erfaßt. Nach Beendigung des Versuches werden die narkotisierten Tiere schmerzfrei durch systemische Injektion von T61 exzitationsfrei eingeschläfert. Zur Auswertung werden die Ergebnisse jeweils von 8 behandelten mit 8 unbehandelten Tieren (Kontrollgruppe) verglichen (Angaben in Prozenten).

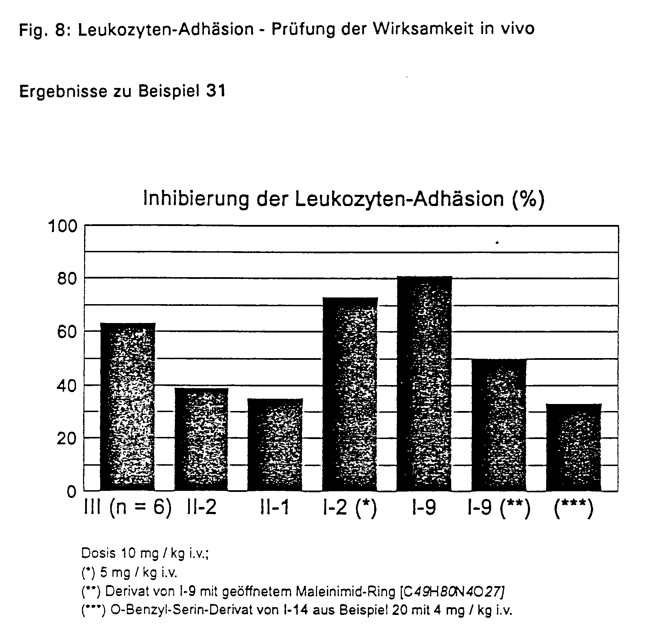

Die Ergebnisse für die Verbindungen II-2, II-1, I-2, I-9 und für Derivate von I-9 und 1-14 sind in Fig. 8 dargestellt.

**Tabelle 1.**

| Inhibierung der HL60 Zelladhäsion an lösliche, rekombinante Adhäsionsmoleküle. | | |
|---|---|---|
| Verbindung | IC₅₀ E-Selektin (mM) | IC₅₀ P-Selektin (mM) |
| I-2 | > 0.80 | 0.01 |
| I-6 | 1.20 | n.b. |
| I-7 | 1.15 | 1.50 |
| I-9 | 0.28 | 0.33 |
| I-10 | 0.39 | 0.40 |
| I-11 | 0.59 | 0.60 |
| I-12 | >0.80 | >0.80 |
| I-13 | 0.02 | 0.10 |
| I-16 | 0.65 | 0.90 |
| II-1 | 0.38 | >0.80 |
| II-2 | 0.13 | 0.14 |
| II-3 | 0.40 | > 1.0 |
| III (n = 6) | 1.0 | 2.0 |

## Patentansprüche

1. Verbindung der Formel I,
Z-Y-(CH₂)ₙ-[NH(CO)]ₚ-R² I,
worin bedeuten
Z -Sialyl-Lewis-X oder Sialyl-Lewis-A
Y Sauerstoff oder NH(CO), das in I Position mit Z verknüpft ist, und
R² einen Aminosäure- oder Oligopeptidrest von bis zu 6 Aminosäuren, einen lipophilen Rest aus aliphatischen oder cycloaliphatischen Bausteinen, -(CH₂)₂- COOH, oder
eine Kombination aus aliphatischen und heterozyklischen Bausteinen oder
einen Triphenylmethanfarbstoff, wobei
für Y = Sauerstoff
p 1 und
n eine ganze Zahl von 2 bis 10 und
für Y = NH(CO) und p = 0
n eine ganze Zahl von 0 bis 10,
für Y = NH(CO) und p = 1
n eine ganze Zahl von 1 bis 10 bedeuten und die Verbindung ein definiertes Molekulargewicht aufweist.

2. Verbindung nach Anspruch 1, **dadurch gekennzeichnet, daß** R2 eine Gruppe der Formel IA, 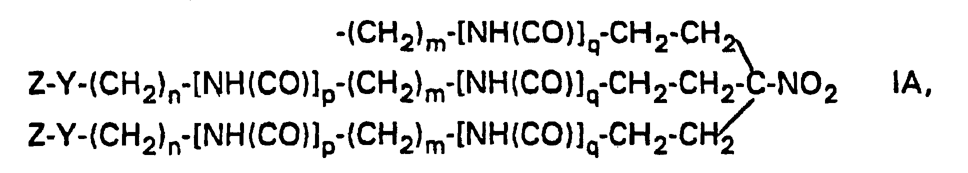 wobei
m eine ganze Zahl von 0 bis 10 ist und
p und q 0 oder 1 sein können mit der Maßgabe, daß
für m = 0 entweder p oder q gleich 0 ist.

3. Verbindung nach Anspruchl, **dadurch gekennzeichnet, daß**
R² -CH₂NH(CO)CH₂NH₂ bedeutet.

4. Verbindung nach Anspruch 1, **dadurch gekennzeichnet, daß**
R² -CH[(S)-CH₃]NHCOCH[(S)-CH₂CO₂H]NHCO-CH₂NHCOCH[(S)-(CH₂)₃NH(C=NH)NH₂]NH₂ bedeutet.

5. Verbindung nach Anspruch1, **dadurch gekennzeichnet, daß**
R² -(CH₂)₆-NH₂ bedeutet.

6. Verbindung nach Anspruch 1, **dadurch gekennzeichnet, daß**
R² -CH(NH₂)-(CH₂)₂-COOH bedeutet.

7. Verbindung nach Anspruch 1, **dadurch gekennzeichnet, daß**
R² -(CH₂)₂-COOH bedeutet.

8. Verbindung nach Anspruch 1, **dadurch gekennzeichnet, daß**
R² -CH(NHZ)CH₂OBn ist, wobei
Z Benzyloxycarbonyl und
Bn Benzyl bedeuten.

9. Verbindung nach Anspruch 1, **dadurch gekennzeichnet, daß**
Y Sauerstoff,
n 6,
p 1 und
R² 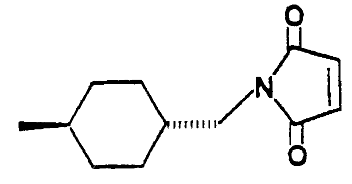
bedeuten.

10. Verbindung nach Anspruch 1, **dadurch gekennzeichnet, daß**
Y Sauerstoff,
n 6,
p 1 und
R² 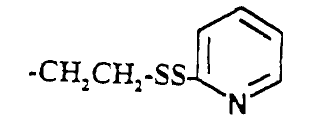
bedeuten.

11. Verbindung nach Anspruch 1, **dadurch gekennzeichnet, daß**
Y Sauerstoff,
n 6,
p 1 und
R² 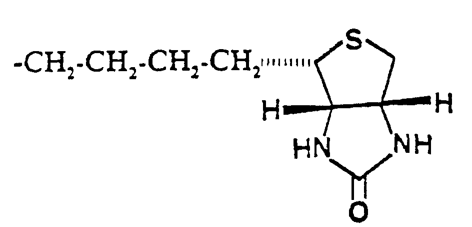
bedeuten.

12. Verbindung nach Anspruch 1, **dadurch gekennzeichnet, daß**
Y Sauerstoff,
n 6,
p 1 und
R² 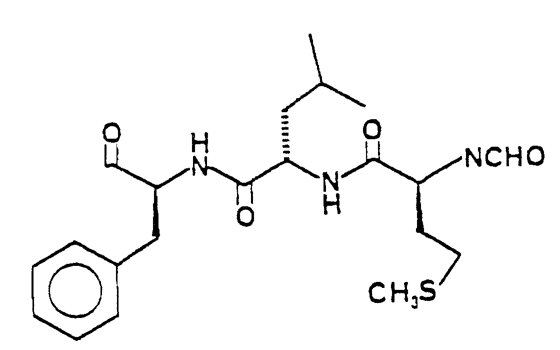
bedeuten.

13. Verbindung nach Anspruch 1, **dadurch gekennzeichnet, daß**
Y Sauerstoff,
n 6,
p 1 und
R² 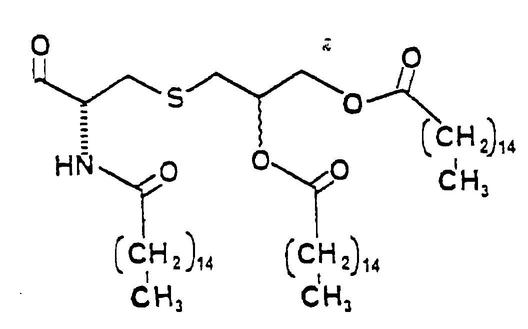
bedeuten.

14. Verbindung nach Anspruch 1, **dadurch gekennzeichnet, daß**
Y Sauerstoff,
n 6,
p 1 und
R² 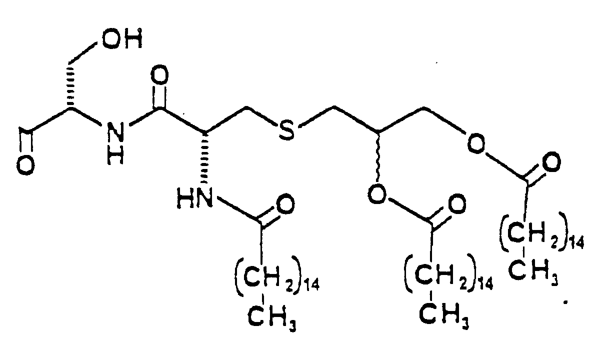
bedeuten.

15. Verbindung nach Anspruch 1, **dadurch gekennzeichnet, daß**
Y Sauerstoff,
n 6,
p 1 und
R² 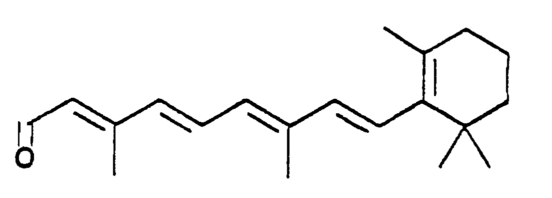
bedeuten.

16. Verbindung nach Anspruch 1, **dadurch gekennzeichnet, daß**
Y Sauerstoff,
n 6,
p 1 und
R² 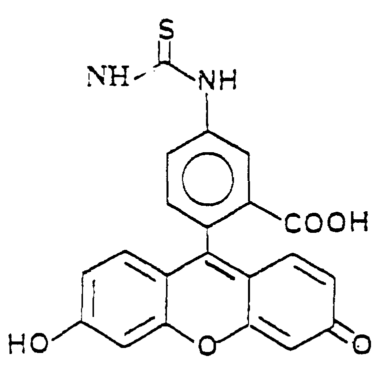
bedeuten.

17. Verfahren zur Herstellung einer Verbindung gemäß einem der Ansprüche 1 bis 16, **dadurch gekennzeichnet, daß** eine Verbindung der Formel III,
Z-O-(CH₂)ₙ-NH₂ III,
eine Verbindung der Formel IV,
Z-NH₂ IV,
oder eine Verbindung der Formel V,
Z-NH(CO)-(CH₂)ₙ-NH₂ , V,
in welchen Z und n die genannten Bedeutungen haben, mit einer Verbindung der Formel VI,
X(CO)R2 VI,
in welcher
X Hydroxyl oder eine carboxylaktivierende Abgangsgruppe und
R² die genannten Bedeutungen hat,
umgesetzt wird, wobei das verzweigte Tetrasaccharid Z der Vorstufen III, IV oder V teilweise in geschützter Form, bevorzugt aber in ungeschüzter Form eingesetzt wird.

18. Verfahren nach Anspruch 17, **dadurch gekennzeichnet, daß** X in Verbindung IV eine O-Succinimidylgruppe ist.

19. Verfahren nach Anspruch 17 oder 18, **dadurch gekennzeichnet, daß** als Lösungsmittel Pyridin eingesetzt wird.

20. Verfahren nach Anspruch 17 oder 18, **dadurch gekennzeichnet, daß** als Lösungsmittel N,N-Dimethylformamid eingesetzt wird.

21. Verfahren nach einem der Ansprüche 17 bis 20 zur Herstellung einer Verbindung gemäß Anspruch 1, **dadurch gekennzeichnet, daß** die Sialinsäure im Tetrasaccharidrest Z von Vorstufe III, IV oder V in der Lactonform vorliegt.

22. Verfahren nach einem der Ansprüche 17 bis 21 zur Herstellung einer Verbindung gemäß Anspruch 2, **dadurch gekennzeichnet, daß** R² in Verbindung VI eine Gruppe der Formel IB, 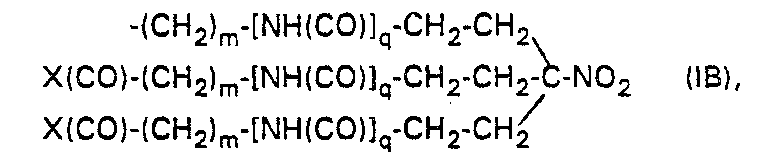 bedeutet, wobei die Variablen m und q die in Anspruch 4 definierten Bedeutungen haben.

23. Arzneimittel enthaltend eine Verbindung gemäß einem der Ansprüche 1 bis 16 und gegebenenfalls pharmazeutische Hilfsstoffe.

24. Verwendung einer Verbindung gemäß einem der Ansprüche 1 bis 16 für die Herstellung eines Arzneimittels zur Vorbeugung oder Heilung von Krankheiten, die durch eine vermehrte Zell-Zell-Adhäsion hervorgerufen werden.

25. Verwendung einer Verbindung gemäß einem der Ansprüche 1 bis 16 für die Herstellung eines Mittels zur Diagnose von Krankheiten, die mit einer vermehrten Zell-Zell-Adhäsion einhergehen.

26. Verwendung einer Verbindung gemäß einem der Ansprüche 1 bis 16 zur Herstellung eines synthetischen Vaccinums.

## Claims

1. A compound of formula I,
Z-Y-(CH₂)ₙ-[NH(CO)]ₚ-R² I,
wherein
Z is a sialyl Lewis X or sialyl Lewis A,
Y oxygen or NH(CO) linked to Z in 1-position, and
R² is an amino acid or oligopeptide residue of up to 6 amino acids,
a liophilic residue of aliphatic or cycloaliphatic moieties, -(CH₂)₂-COOH, or
a combination of aliphatic and heterocyclic moieties or a triphenyl methane dye, where
for Y = oxygen
p is 1 and
n is an integer of 2 to 10, and
for Y = NH(CO) and p =0
n is an integer of 0 to 10,
for Y = NH(CO) and p = 1
n is an integer of 1 to 10, and the compound has a defined molecular weight.

2. The compound according to claim 1, **characterized in that** R² is a group of formula IA, 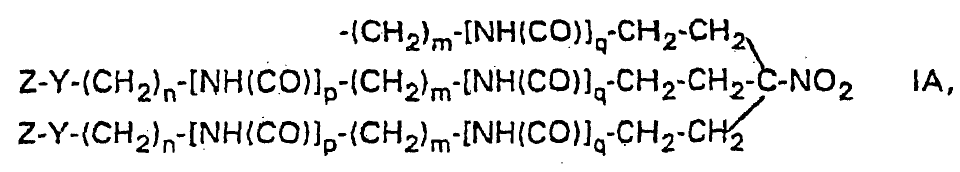 where
m is an integer of 0 to 10, and
p and q may be 0 or 1, with the proviso that
for m = 0 either p or q is equal to 0.

3. The compound according to claim 1, **characterized in that**
R² is -CH₂NH(CO)CH₂NH₂.

4. The compound according to claim 1, **characterized in that**
R² is -CH[(S)-CH₃]NHCOCH[(S)-CH₂CO₂H]NHCO-CH₂NHCOCH[(S)-(CH₂)₃)NH(C=NH)NH₂]NH₂.

5. The compound according to claim 1, **characterized in that**
R² is -(CH₂)₆-NH₂.

6. The compound according to claim 1, **characterized in that**
R² is -CH(NH₂)-(CH₂)₂-COOH.

7. The compound according to claim 1, **characterized in that**
R² is -(CH₂)₂-COOH.

8. The compound according to claim 1, **characterized in that**
R² is -CH(NHZ)CH₂OBn, where
Z means benzyloxycarbonyl and
Bn benzyl.

9. The compound according to claim 1, **characterized in that**
Y is oxygen,
n is 6,
p is 1 and
R²

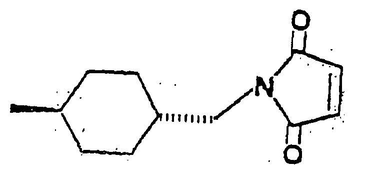

10. The compound according to claim 1, **characterized in that**
Y is oxygen
n is 6,
p is 1 and
R²

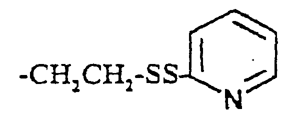

11. The compound according to claim 1, **characterized in that**
Y is oxygen,
n is 6,
p is 1 and
R²

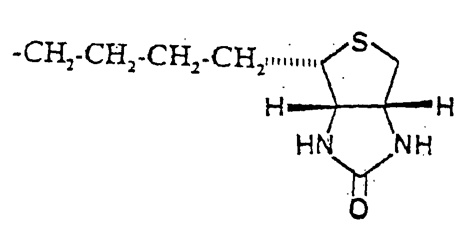

12. The compound according to claim 1, **characterized in that**
Y is oxygen,
n is 6,
p is 1 and
R²

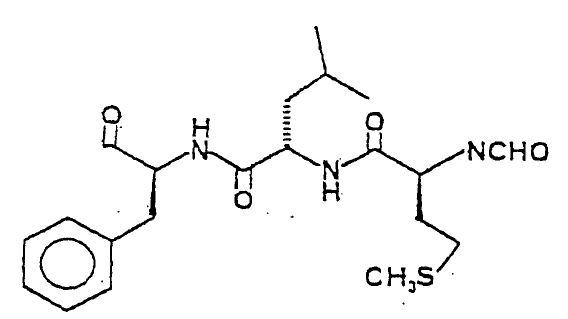

13. The compound according to claim 1, **characterized in that**
Y is oxygen,
n is 6,
p is 1 and
R²

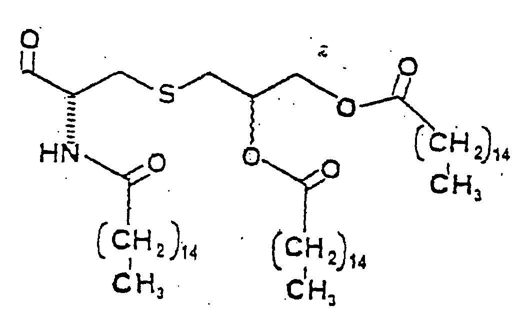

14. The compound according to claim 1, **characterized in that**
Y is oxygen,
n is 6,
p is 1 and
R²

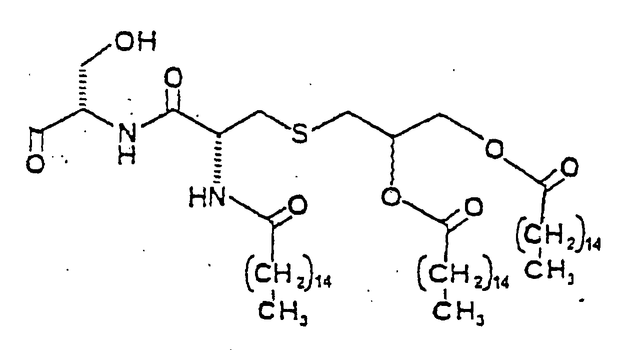

15. The compound according to claim 1, **characterized in that**
Y is oxygen,
n is 6,
p is 1, and
R²

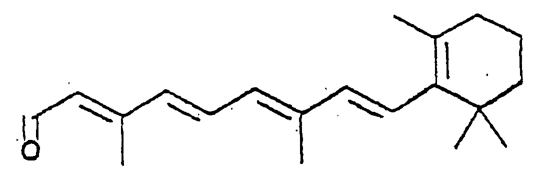

16. The compound according to claim 1, **characterized in that**
Y is oxygen,
n is 6,
p is 1 and
R²

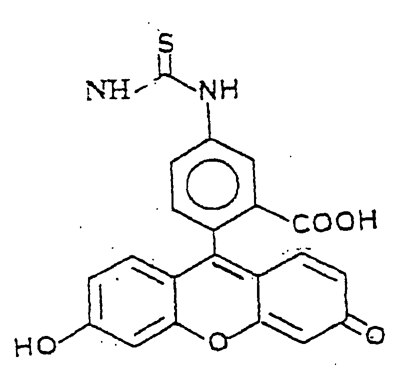

17. A method for preparing a compound according to any one of claims 1 to 16, **characterized in that** a compound of formula III,
Z-O-(CH₂)ₙ-NH₂ III,
a compound of formula IV,
Z-NH₂ IV,
or a compound of formula V,
Z-NH(CO)-(CH₂)ₙ-NH₂ V,
in which Z and n have the said meanings, is reacted with a compound of formula VI,
X(CO)R2 VI,
in which
X is hydroxyl or a carboxyl-activated leaving group, and
R² has the said meanings,
wherein the branched tetrasaccharide Z of precursor III, IV or V is partly used in protected form, but preferably in unprotected form.

18. The method according to claim 17, **characterized in that** X in combination with IV is an O-succinimidyl group.

19. The method according to claim 17 or 18, **characterized in that** pyridine is used as solvent.

20. The method according to claim 17 or 18, **characterized in that** N,N-dimethyl formamide is used as solvent.

21. The method according to any one of claims 17 to 20 for preparing a compound according to claim 1, **characterized in that** the sialic acid in the tetrasaccharide residue Z of precursor III, IV or V is present in the form of lactone.

22. The method according to any one of claims 17 to 21 for preparing a compound according to claim 2, **characterized in that** R² in compound VI means a group of formula IB, 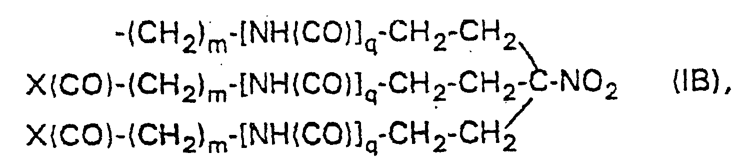 where the variables m and q have the meanings defined in claim 4.

23. A drug comprising a compound according to any one of claims 1 to 16 and, optionally, pharmaceutical adjuvants.

24. Use of a compound according to any one of claims 1 to 16 for preparing a drug for prophylaxis or for healing diseases caused by an increased cell-cell adhesion.

25. Use of a compound according to any one of claims 1 to 16 for preparing an agent for diagnosing diseases accompanied by an increased cell-cell adhesion.

26. Use of a compound according to any one of claims 1 to 16 for preparing a synthetic vaccinum.

## Revendications

1. Composé de formule I,
Z-Y-(CH₂)ₙ-[NH(CO)]ₚ-R² I,
dans laquelle
Z représente un sialyl-Lewis-X ou un sialyl-Lewis-A,
Y est l'oxygène ou NH(CO), relié à Z en position 1, et
R² représente un radical acide aminé ou oligopeptide comportant jusqu'à 6 acides aminés, un radical lipophile composé d'éléments aliphatiques ou cycloaliphatiques, -(CH₂)₂-COOH ou une combinaison d'éléments aliphatiques et hétérocycliques ou un colorant triphénylméthane, où
si Y = oxygène
p vaut 1 et
n est un entier compris entre 2 et 10, et
si Y = NH(CO) et p = 0
n est un entier compris entre 0 et 10,
si Y = NH(CO) et p = 1
n est un entier compris entre 1 et 10, et le composé présente une masse moléculaire définie.

2. Composé selon la revendication 1, **caractérisé en ce que** R² représente un groupe de formule IA 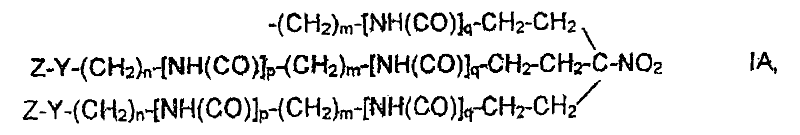 dans laquelle
m est un entier compris entre 0 et 10, et
p et q peuvent valoir 0 ou 1, à la condition que
si m = 0, p ou q est égal à zéro.

3. Composé selon la revendication 1, **caractérisé en ce que** R² représente -CH₂NH(CO)CH₂NH₂.

4. Composé selon la revendication 1, **caractérisé en ce que** R² représente
-CH[(S)-CH₃]NHCOCH[(S)-CH₂CO₂H]NHCO-CH₂NHCOCH[(S)-(CH₂)₃NH(C=NH)NH₂]NH₂.

5. Composé selon la revendication 1, **caractérisé en ce que** R² représente -(CH₂)₆-NH₂.

6. Composé selon la revendication 1, **caractérisé en ce que** R² représente -CH(NH₂)-(CH₂)₂-COOH.

7. Composé selon la revendication 1, **caractérisé en ce que** R² représente -(CH₂)₂-COOH.

8. Composé selon la revendication 1, **caractérisé en ce que** R² représente -CH(NHZ)CH₂OBn, où
Z est un benzyloxycarbonyle et
Bn représente un benzyle.

9. Composé selon la revendication 1, **caractérisé en ce que**
Y est l'oxygène
n vaut 6
p vaut 1, et
R² représente

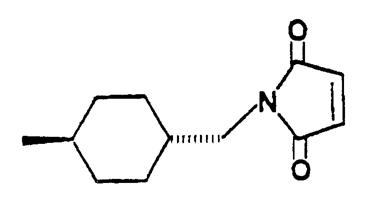

10. Composé selon la revendication 1, **caractérisé en ce que**
Y est l'oxygène
n vaut 6
p vaut 1, et
R² représente

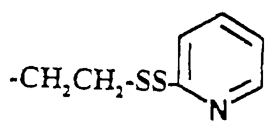

11. Composé selon la revendication 1, **caractérisé en ce que**
Y est l'oxygène
n vaut 6
p vaut 1, et
R² représente

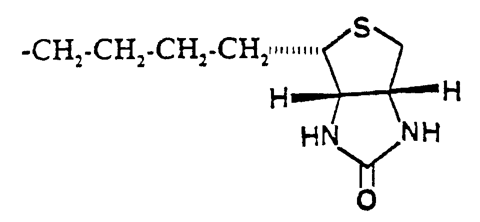

12. Composé selon la revendication 1, **caractérisé en ce que**
Y est l'oxygène
n vaut 6
p vaut 1, et
R² représente

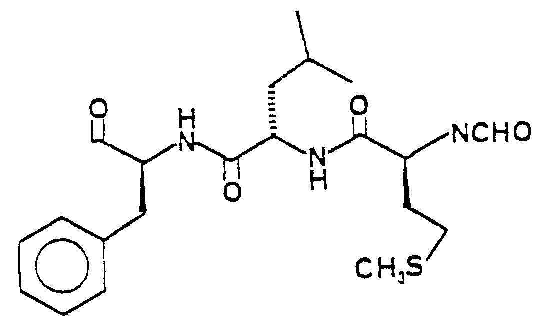

13. Composé selon la revendication 1, **caractérisé en ce que**
Y est l'oxygène
n vaut 6
p vaut 1, et
R² représente

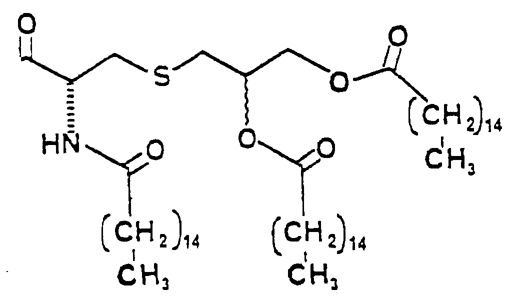

14. Composé selon la revendication 1, **caractérisé en ce que**
Y est l'oxgène
n vaut 6
p vaut 1, et
R² représente

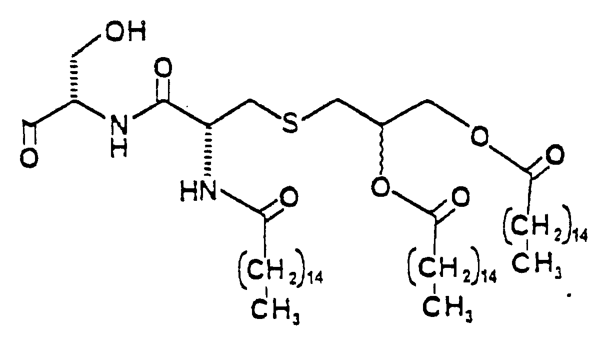

15. Composé selon la revendication 1, **caractérisé en ce que**
Y est l'oxygène
n vaut 6
p vaut 1, et
R² représente

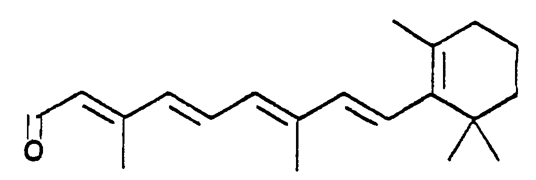

16. Composé selon la revendication 1, **caractérisé en ce que**
Y est l'oxygène
n vaut 6
p vaut 1, et
R² représente

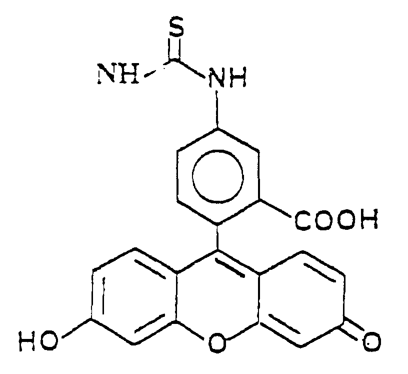

17. Procédé de préparation d'un composé selon l'une des revendications 1 à 16, **caractérisé en ce qu'**un composé de formule III,
Z-O-(CH₂)ₙ-NH₂ III,
un composé de formule IV,
Z-NH₂ IV,
ou un composé de formule V,
Z-NH(CO)-(CH₂)ₙ-NH₂ V,
où Z et n ont les significations nommées, est mis à réagir avec un composé de formule VI
X(CO)R² VI,
dans laquelle
X est un hydroxyle ou un groupe partant activant un carboxyle et
R² a les significations nommées,
le tétraholoside ramifié Z des précurseurs III, IV ou V étant utilisé partiellement sous une forme protégée, de préférence cependant sous une forme non protégée.

18. Procédé selon la revendication 17, **caractérisé en ce que** le groupement X du composé VI est un groupe O-succinimidyle

19. Procédé selon la revendication 17 ou 18, **caractérisé en ce que** l'on utilise de la pyridine comme solvant.

20. Procédé selon la revendication 17 ou 18, **caractérisé en ce que** l'on utilise du N,N-diméthylformamide comme solvant.

21. Procédé selon l'une des revendications 17 à 20 pour préparer un composé selon la revendication 1, **caractérisé en ce que** l'acide sialique du radical tétraholoside Z du précurseur III, IV ou V se présente sous forme de lactone.

22. Procédé selon l'une des revendications 17 à 21 pour préparer un composé selon la revendication 2, **caractérisé en ce que** le radical R² du composé VI représente un groupe de formule IB, 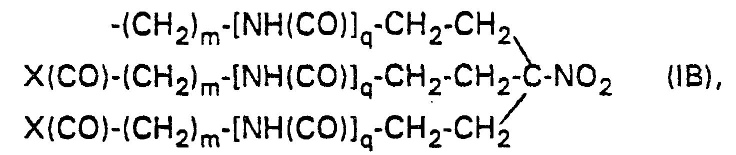 les variables m et q ayant les significations définies dans la revendication 4.

23. Médicament contenant un composé selon l'une des revendications 1 à 16, et éventuellement des auxillaires pharmaceutiques.

24. Utilisation d'un composé selon l'une des revendications 1 à 16 pour la préparation d'un médicament destiné à prévenir ou guérir des maladies qui sont causées par une multiplication de l'adhesion cellule-cellule.

25. Utilisation d'un composé selon l'une des revendications 1 à 16 pour la préparation d'un agent permettant le diagnostic de maladies qui s'accompagnent de la multiplication de l'adhésion cellule-cellule.

26. Utilisation d'un composé selon l'une des revendications 1 à 16 pour la préparation d'un vaccin synthétique.
